# EUROPEAN PATENT SPECIFICATION

(11) **EP 1 305 422 B1**
(45) Date of publication and mention of the grant of the patent: **18.05.2011**
(21) Application number: 01959520.6
(22) Date of filing: 03.08.2001
(51) Int. Cl.: C12N 15/31, C07K 14/24, C07K 14/26, C12P 7/60

(54) **2,5-DKG PERMEASES**
2,5-DKG-PERMEASEN
PERMEASES 2,5-DKG

(30) Priority: 04.08.2000 US 633294; 29.09.2000 US 677032
(43) Date of publication of application: 02.05.2003
(73) Proprietor: Microgenomics, Inc., Carlsbad, CA 92008 (US); Danisco US Inc., Palo Alto, CA 94304 (US)
(72) Inventor: DARTOIS, Veronique, A., San Diego, CA 92122 (US); HOCH, James, A., La Jolla, CA 92037 (US); VALLE, Fernando, Burlingame, CA 94010 (US); KUMAR, Manoj, Fremont, CA 94555 (US)
(74) Representative: Kiddle, Simon John
(86) International application number: PCT/US2001/024507
(87) International publication number: WO 2002/012468

(56) References cited:
- WO-A-00/22170
- WO-A-97/25432
- DATABASE EMBL [Online] EMBL; 2 July 1998 (1998-07-02) HAMOOD ET AL.: "Pseudomonas aeruginosa PtxR (ptxR) and PtxS (ptxS) genes, complete cds and ketogluconate utilization operon gene, complete sequence" Database accession no. AF012100 XP002204795 -& SWANSON B L ET AL.: "Characterization of the 2-ketogluconate utilization operon in Pseudomonas aeruginosa PAO1" MOLECULAR MICROBIOLOGY, vol. 37, no. 3, August 2000 (2000-08), pages 561-573, XP002204788
- ANDERSON S ET AL: "Production of 2-keto-L-gulonate, an intermediate in L-ascorbate synthesis, by a genetically modified Erwinia herbicola" SCIENCE, vol. 230, no. 4722, 11 October 1985 (1985-10-11), pages 144-149, XP002031456 ISSN: 0036-8075
- BOUDRANT J: "Microbial processes for ascorbic acid biosynthesis: a review" ENZYME AND MICROBIAL TECHNOLOGY, vol. 12, no. 5, 1 May 1990 (1990-05-01), pages 322-329, XP000646789 ISSN: 0141-0229
- SAITO Y ET AL.: "Direct fermentation of 2-Keto-L-gulonic acid in recombinant Gluconobacter oxydans." BIOTECHNOLOGY AND BIOENGINEERING, vol. 58, no. 2-3, 20 April 1998 (1998-04-20), pages 309-315, XP002204789 ISSN: 0006-3592
- IZU H ET AL.: "Characterization of the gntT gene encoding a high-affinity gluconate permease in Escherichia coli" GENE, vol. 199, no. 1-2, 15 October 1997 (1997-10-15), pages 203-210, XP004126382 ISSN: 0378-1119
- BAUSCH C ET AL.: "Sequence analysis of the GntII (subsidiary) system for gluconate metabolism reveals a novel pathway for L-idonic acid catabolism in Escherichia coli." JOURNAL OF BACTERIOLOGY, vol. 180, no. 14, July 1998 (1998-07), pages 3704-3710, XP002204790 ISSN: 0021-9193
- KLEMM P ET AL.: "The gntP gene of Escherichia coli involved in gluconate uptake." JOURNAL OF BACTERIOLOGY, vol. 178, no. 1, 1996, pages 61-67, XP002204791 ISSN: 0021-9193
- PEEKHAUS N ET AL.: "Characterization of a novel transporter family that includes multiple Escherichia coli gluconate transporters and their homologues." FEMS MICROBIOLOGY LETTERS, vol. 147, no. 2, 1997, pages 233-238, XP002204792 ISSN: 0378-1097
- CHOTANI G ET AL.: "The commercial production of chemicals using pathway engineering." BIOCHIMICA ET BIOPHYSICA ACTA, vol. 1543, no. 2, December 2000 (2000-12), pages 434-455, XP004279117 ISSN: 0006-3002
- YEW W S ET AL.: "Utilization of L-ascorbate by Escherichia coli K-12: Assignments of functions to products of the yjf-sga and yia-sgb operons." JOURNAL OF BACTERIOLOGY, vol. 184, no. 1, January 2002 (2002-01), pages 302-306, XP002204794 ISSN: 0021-9193

## Description

This invention was made in part with U.S. Government support under Cooperative Agreement 70NANB5H1138 and ATP NIST project Identification Number 1995-05-0007E. The U.S. Government has certain rights in this invention.

### BACKGROUND OF THE INVENTION

### FIELD OF THE INVENTION

The present invention relates generally to microbial transporter proteins and, more specifically, to novel 2,5-diketo-D-gluconic acid (2,5-DKG) permeases.

### BACKGROUND INFORMATION

Adequate intake of ascorbic acid, or vitamin C, is recognized as an important factor in maintaining health. To ensure adequate intake of ascorbic acid, the chemical is now added to many foods, drinks and cosmetic products, and is also sold as a direct vitamin supplement. To meet the commercial demand for ascorbic acid, there is a need to develop more efficient processes for its production.

Although there are a number of alternative methods of producing ascorbic acid, one of the least expensive and most ecologically sound methods is biofermentation. Bacterial strains have now been engineered to express all of the enzymes required for the stepwise conversion of an inexpensive sugar source, such as D-glucose, to a stable precursor of ascorbic acid, 2-keto-L-gulonic acid (2-KLG) (see U.S. Patent No. 5,032,514 and references therein). 2-KLG can be readily converted to ascorbic acid by chemical or enzymatic procedures.

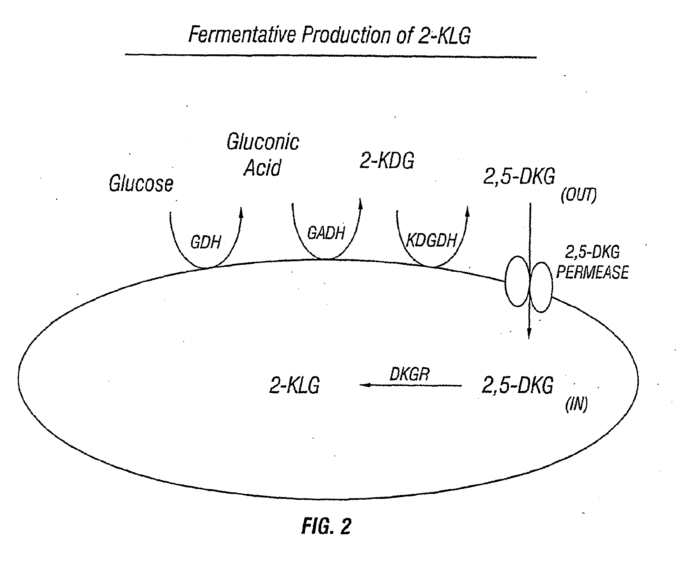

Figure 2 shows schematically the enzymatic reactions that take place in the bioconversion of D-glucose to 2-KLG. As shown in Figure 2, the enzymatic reactions that lead from D-glucose, to D-gluconic acid, to 2-keto-D-gluconic acid (2-KDG), to 2,5-diketo-D-gluconic acid (2,5-DKG), take place at the surface of the bacterial cell. 2,5-DKG must then enter the cell in order for its enzymatic conversion to 2-KLG.

Hamood et al., 1996, Mol. Microbiol. Vol. 21 (1), pp 97-110, describes a *Pseudomonas aeruginosa* gene encoding a 2-KDG transporter; WO00122170 describes a metabolic pathway involved in the conversion of 2-keto-L-gluconate to ascorbic acid in *Klebsiella oxytoca.*

Much effort has been expended in increasing the efficiency of the enzymatic reactions involved in 2-KLG production. For example, U.S. Patent No. 5,032,514 describes methods for increasing 2-KLG production by reducing metabolic diversion of 2,5-DKG to products other than 2-KLG.

Increasing uptake of 2,5-DKG by a bacterial strain suitable for biofermentation could be advantageous in increasing 2-KLG production. Expressing additional copies of an endogenous 2,5-DKG permease, or expressing an exogenous 2,5-DKG permease with superior properties, could increase uptake of 2,5-DKG. However, to date, no 2,5-DKG permease has been identified or characterized that could be used in this manner.

Therefore, there exists a need to identify and characterize nucleic acid molecules encoding 2,5-DKG permeases, so that permeases with advantageous properties can be used in the commercial production of ascorbic acid and in other important applications. The present invention satisfies this need and provides related advantages as well.

### SUMMARY OF THE INVENTION

The invention provides an isolated nucleic acid molecule as defined in the claims encoding a polypeptide which has 2,5-DKG permease activity. In one embodiment, the isolated nucleic acid molecule contains a nucleotide sequence having at least 80% identity to a nucleotide sequence selected from the group consisting of SEQ ID NOS:1, 3, 5, 7, and 9. In another embodiment, the isolated nucleic acid molecule contains a nucleotide sequence which encodes a polypeptide having at least 80% identity to an amino acid sequence selected from the group consisting of SEQ ID NOS:2, 4, 6, 8, and 10.

Also provided are vectors and cells containing isolated nucleic acid molecules as defined in the claims encoding polypeptides having 2,5-KDG permease activity. In one embodiment, the cells are bacterial cells selected from the genera Pantoea and *Klebsiella*.

The invention also provides methods of identifying and isolating nucleic acid molecules encoding polypeptides which have 2,5-DKG permease activity. Also provided are methods of enhancing 2-KLG production, by expressing the nucleic acid molecules of the invention in suitable bacterial cells.

Further provided are isolated polypeptides as defined in the claims having 2,5-DKG permease activity. The invention also provides antibodies specific for such polypeptides.

### BRIEF DESCRIPTION OF THE DRAWINGS

Figure 1A and 1B shows an alignment of the amino acid sequences of the 2,5-DKG permeases designated YiaX2 (SEQ ID NO:12); PE1 (SEQ ID NO:2); PE6 (SEQ ID NO:4); prmA (SEQ ID NO:8); prmB (SEQ ID NO:10) and PK1 (SEQ ID NO:6). SEQ ID NO: 12 is included for comparative purposes only.
Figure 2 shows the biosynthetic pathway from glucose to 2-KLG in a bacterial strain suitable for biofermentation.
Figure 3 shows the metabolic selection strategy used to identify novel 2,5-DKG permeases.

### DETAILED DESCRIPTION OF THE INVENTION

The invention provides novel nucleic acid molecules as defined in the claims encoding polypeptides having 2,5-DKG permease activity, and related products and methods. The molecules of the invention can advantageously be used to increase the efficiency of 2-KLG bioproduction, and thus to lower the cost of commercial ascorbic acid production.

Naturally occurring 2,5-DKG permeases are polypeptides localized to the cytoplasmic membrane of microorganisms, which are predicted, using commercially available topology prediction programs, to contain about 10 to 12 transmembrane domains. Each transmembrane spanning segment is about 20 amino acids in length, with the intracellular and extracellular loops ranging from about 2 to about 83 amino acids in length. Generally, the loop between the fifth and sixth transmembrane domain spanning segments is larger than the other loops.

Naturally occurring 2,5-DKG permeases are typically about 350-550 amino acids in length, such as about 400-450 amino acids in length, and particularly about 425-440 amino acids in length.

The nucleotide sequences encoding six exemplary 2,5-DKG permeases are set forth as follows, with the designation and organismal source of the molecule indicated in parentheses: SEQ ID NO:1 (PE1 from an environmental source); SEQ ID NO:3 (PE6 from an environmental source); SEQ ID NO:5 (PK1 from *Klebsiella oxytoca*); SEQ ID NO:7 (prmA from *Pantoea citrea*); SEQ ID NO:9 (prmB from Pantoea citrea); and comparative SEQ ID NO:11 (YiaX2 from *Klebsiella oxytoca*). The corresponding encoded 2,5-DKG permease amino acid sequences are set forth as SEQ ID NO:2 (PE1); SEQ ID NO:4 (PE6); SEQ ID NO:6 (PK1); SEQ ID NO:8 (prmA); SEQ ID NO:10 (prmB); and comparative SEQ ID NO:12 (YiaX2).

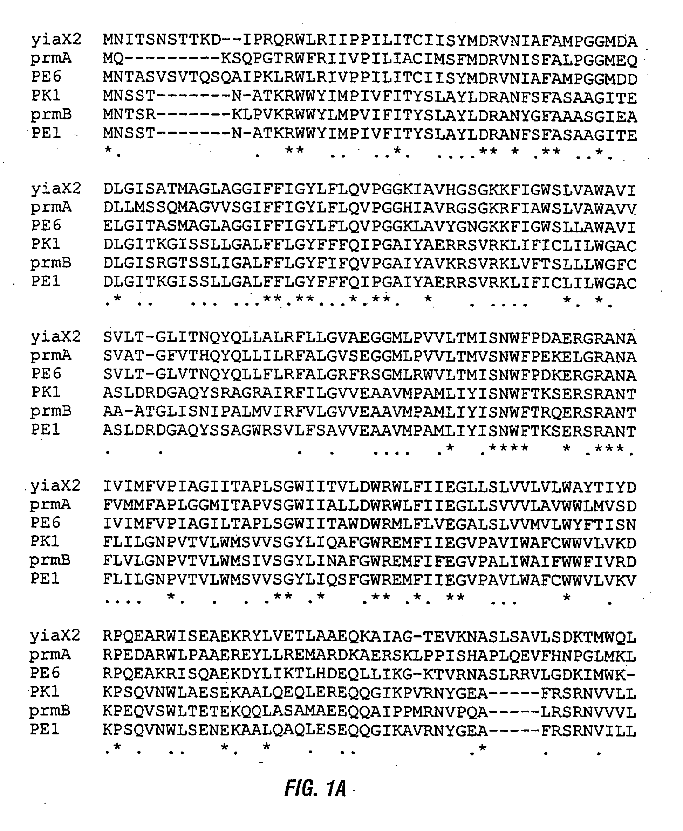

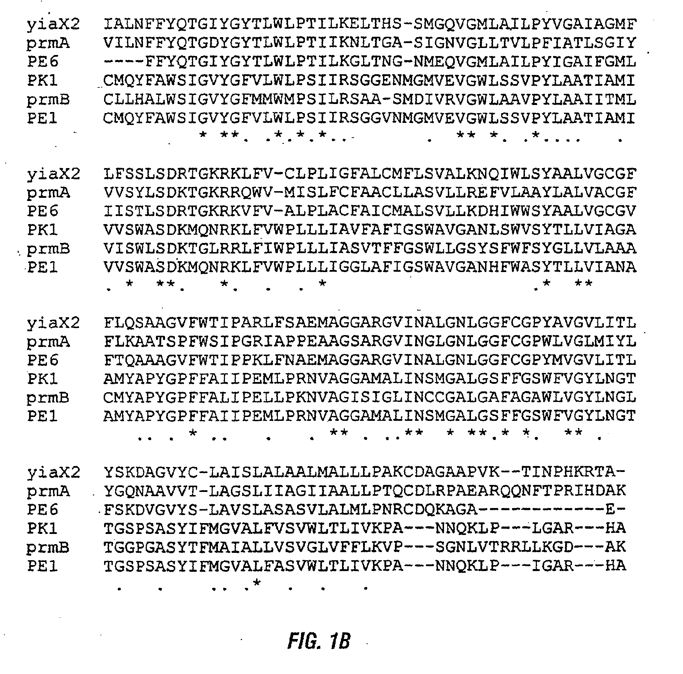

2,5-DKG permeases from different microorganisms exhibit extensive amino acid sequence relatedness over their entire length, as is evidenced by the six-way sequence alignment shown in Figure 1. The overall identity of the six permeases shown in Figure 1 is about 17%, and the overall similarity, taking into account conservative substitutions, is about 43%.

Based on their predicted topological and sequence similarity, 2,5-DKG permeases disclosed herein can be further subdivided into two structural families. The three 2,5-DKG permeases designated YiaX2, PE6 and PrmA are representative of one family of permeases, sharing about 50% overall identity in a three-way amino acid sequence alignment (YiaX2 is included for comparative purposes only). The three 2,5-DKG permeases designated PK1, PE1 and PrmB are representative of a second family of related permeases, sharing about 60% overall identity in a three-way amino acid sequence alignment.

Naturally occurring 2,5-DKG permeases also exhibit 2,5-DKG permease activity. The term "2,5-DKG permease activity," as used herein, refers to the ability of the polypeptide, when expressed in its native orientation at the cell membrane, to transport 2,5-DKG across the cytoplasmic membrane, in comparison with an unrelated control polypeptide. Such transport can be either unidirectional or bidirectional.

2,5-DKG permease activity can be determined by a variety of methods. For example, 2,5-DKG permease activity can be determined using a metabolic selection assay, as described further in the Example, below. Briefly, a bacterial cell either naturally deficient in 2,5-DKG permease activity, or made deficient in 2,5-DKG permease activity, is identified or produced. As described in the Example, bacterial cells can be made deficient in endogenous 2,5-DKG permease activity by preparing a deletion mutant of one or more endogenous 2,5-DKG permease genes, using the polymerase chain reaction, following methods known in the art. The term "deficient," as used in relation to a cell deficient in 2,5-DKG permease activity, is intended to refer to endogenous 2,5-DKG permease activity that is comparable to, or less than, the endogenous permease activity of a *K. oxytoca* strain deleted in the *yiaX2* gene, such as the strain K. *oxytoca* Δ*yiaX2* [*tkr idnO*], as assessed either by a growth assay or by a 2,5-DKG uptake assay.

A cell useful in a metabolic selection assay to determine 2,5-DKG permease activity of an expressed polypeptide can further naturally be capable of converting intracellular 2,5-DKG to carbon and energy, or made capable of such conversion by recombinant expression of appropriate metabolic enzymes. As described in the Example, a combination of nucleic acid molecules encoding a 2-keto-reductase (*tkr*) and a 5-keto-reductase (*idnO*), from any bacterial species, can be expressed in the cell, which together provide the cell with the ability to catalyze the reduction of 2,5-DKG to gluconic acid. Gluconic acid can then be used by the cell as a carbon and energy source that supports cell growth.

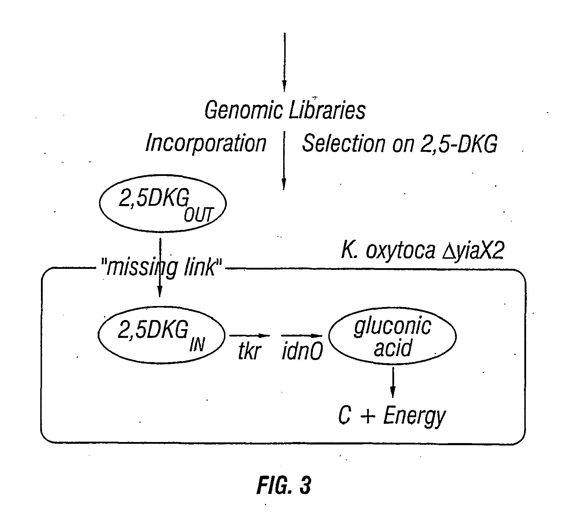

An exemplary bacterial cell suitable for metabolic assays to determine 2,5-DKG permease activity is the strain *K. oxytoca* Δ*yiaX2* [*tkr idnO*] shown in Figure 3 and described in the Example, below. This strain has a deleted *yiaX2* 2,5-DKG permease gene, and also recombinantly expresses the *tkr*/*idnD*/*idnO* operon set forth as SEQ ID NO:13 on a high copy number plasmid. Within SEQ ID NO:13, nucleotides 292-1236 encode a 2-keto-reductase (tkr) (SEQ ID NO:14); nucleotides 1252-2280 encode an idonic acid dehydrogenase (idnD) (SEQ ID NO:15); and nucleotides 2293-3045 encode a 5-keto-reductase (idnO) (SEQ ID NO:16). Alternatively, nucleic acid molecules encoding polypeptides which contain modifications from the amino acid,sequences designated SEQ ID NO:14 or 16, but which retain 2-keto-reductase activity or 5-keto-reductase activity, respectively, can be used in metabolic assays. Exemplary amino acid sequences have at least 60%, such as at least 70%, preferably 80%, 90%, 95% or greater identity to SEQ ID NOS:14 or 16, respectively.

The ability of such a bacterial cell to grow on medium containing 2,5-DKG as the sole carbon source, upon expression of a candidate 2,5-DKG permease, is a measure of the ability of the expressed permease to transport 2,5-DKG into the cell, and is thus a measure of its 2,5-DKG permease activity. Each of SEQ ID NOS:2, 4, 6, 8, 10 and comparative 12 was demonstrated to have 2,5-DKG permease activity, as evidenced by the ability of *K. oxytoca* Δ*yiaX2* [*tkr idnO*] expressing each permease to grow on 2,5-DKG as the sole carbon source.

Likewise, 2,5-DKG permease activity can be determined by measuring uptake of labeled or unlabeled 2,5-DKG. For example, 2,5-DKG can be detectably labeled, such as with a fluorescent or radioactive tag. The ability of a cell or membrane vesicle expressing a 2,5-DKG permease to take up the detectable label when provided with detectably labeled 2,5-DKG, can be determined using detection assays specific for the particular label, which are well known in the art. Likewise, uptake of unlabeled 2,5-DKG can be measured by HPLC or other sensitive detection assay known in the art. Uptake of 2,5-DKG is thus a measure of permease activity. Each of SEQ ID NOS:2, 4, 6, 8, 10 and comparative 12 exhibits 2,5-DKG permease activity as determined by assay of uptake of radiolabeled 2,5-DKG by bacterial cells expressing the recombinant permeases.

Additionally, 2,5-DKG permease activity can be measured in any cell in which 2,5-DKG can be converted to a product, by measuring production of the product in the presence of extracellular 2,5-DKG. For example, in a cell naturally expressing, or recombinantly expressing, a 2,5-DKG reductase, intracellular 2,5-DKG is converted to 2-KLG. The ability of the bacterial cell to produce 2-KLG when provided with extracellular 2,5-DKG, upon expression of a 2,5-DKG permease, is a measure of the ability of the expressed permease to transport 2,5-DKG into the cell, and is thus a measure of its 2,5-DKG permease activity. Intracellular 2-KLG can be detected, for example, using HPLC or other sensitive detection methods known in the art. Other metabolic products of 2,5-DKG can also be detected, by similar methods.

It will be appreciated that a variety of alternative assays can be used to determine 2,5-DKG permease activity. For instance, the change in pH across a cell or vesicle membrane as 2,5-DKG, an acid, is transported across the membrane can be detected. Similarly, a decrease over time in extracellular 2,5-DKG can be determined.

Accordingly, using any of the activity assays described herein, those skilled in the art can distinguish between a polypeptide having 2,5-DKG permease activity, and a polypeptide not having such activity.

A 2,5-DKG permease of the invention can selectively transport 2,5-DKG. As used herein in relation to transport activity, the term "selective" refers to preferential transport of 2,5-DKG rather than 2-KLG into or out of the cell. A permease that selectively transports 2,5-DKG will transport 2,5-DKG at least 2-fold, such as at least 5-fold, including greater than 10-fold more efficiently than it transports 2-KLG. A permease that selectively transports 2,5-DKG is particularly advantageous in applications where it is desirable to increase intracellular production of 2-KLG, such as in the commercial production of ascorbic acid. In particular, employing a permease that selectively transports 2,5-DKG prevents intracellular 2-KLG from competing with extracellular 2,5-DKG for permease-mediated transport through the membrane, and increases the overall efficiency of intracellular 2-KLG production.

It will be appreciated that the assays described above for determining 2,5-DKG permease activity can be modified to simultaneously, or separately, determine 2-KLG permease activity. For example, a metabolic assay can be designed in which a bacterial cell can convert either intracellular 2,5-DKG or 2-KLG to carbon and energy. In such a cell, the relative ability of the cell to grow on 2,5-DKG as the sole carbon source, compared with its ability to grow on 2-KLG as the sole carbon source, is a measure of the ability of the expressed permease to selectively transport 2,5-DKG. Using such an assay, it was determined that the 2,5-DKG permeases designated YiaX2, PE1, PE6, prmA and prmB are non-selective for 2,5-DKG, as they also efficiently catalyze the transport of 2-KLG, as *K. oxytoca* Δ*yiaX2* [*tkr idnO*] cells expressing such permeases grow well on either 2,5-DKG or 2-KLG (YiaX2 is included for comparative purposes only). In contrast, PK1 selectively transports 2,5-DKG, and *K. oxytoca ΔyiaX2* [*tkr idnO*] cells expressing PK1 (SEQ ID NO:6) grow on 2,5-DKG but not on 2-KLG as the sole carbon source.

The invention provides an isolated nucleic acid molecule as defined in the claims encoding a polypeptide which has 2,5-DKG permease activity. The nucleic acid molecules of the invention are suitable for a variety of commercial and research applications. For example, one or more of the invention nucleic acid molecules can be expressed in bacterial cells in order to enhance the rate of uptake of 2,5-DKG by the cells. Enhancing uptake of 2,5-DKG has a variety of applications, such as in commercial production of 2,5-DKG itself, or commercial production of any metabolic product of 2,5-DKG. For example, 2,5-DKG uptake is a rate limiting step in the biosynthesis of 2-KLG, which is a stable intermediate in the synthesis of ascorbic acid. 2-KLG can thus be obtained from bacterial cells expressing 2,5-DKG permeases, and converted to ascorbic acid.

Additionally, the invention nucleic acid molecules can be used as probes or to design primers to identify and isolate 2,5-DKG permease homologs from additional species, or as templates for the production of mutant permeases, using methods known in the art and described further below. Such permeases can have advantageous properties compared with the 2,5-DKG permeases disclosed herein as SEQ ID NOS:2, 4, 6, 8, and 10, such as greater enzymatic activity or greater 2,5-DKG selectivity.

The term "isolated," as used herein, is intended to mean that the molecule is altered, by the hand of man, from how it is found in its natural environment. For example, an isolated nucleic acid molecule can be a molecule operatively linked to an exogenous nucleic acid sequence. An isolated nucleic acid molecule can also be a molecule removed from some or all of its normal flanking nucleic acid sequences, such as removed from one or more other genes within the operon in which the nucleic acid molecule is normally found.

Specifically with respect to an isolated nucleic acid molecule containing the nucleotide sequence designated comparative SEQ ID NO:11, or encoding the *yiaX2* polypeptide designated comparative SEQ ID NO:12, the term "isolated" is intended to mean that the nucleic acid molecule does not contain any of the flanking open reading frames (orfs) present in the *K. oxytoca yia* operon, such as the orfs designated *lyxK* and *orf1,* described in WO 00/22170.

An isolated molecule can alternatively, or additionally, be a "substantially pure" molecule, in that the molecule is at least 60%, 70%, 80%, 90 or 95% free from cellular components with which it is naturally associated. An isolated nucleic acid molecule can be in any form, such as in a buffered solution, a suspension, a heterologous cell, a lyophilized powder, or attached to a solid support.

The term "nucleic acid molecule" as used herein refers to a polynucleotide of natural or synthetic origin. A nucleic acid molecule can be single- or double-stranded genomic DNA, cDNA or RNA, and represent either the sense or antisense strand or both. A nucleic acid molecule can thus correspond to the recited sequence, to its complement, or both.

The term "nucleic acid molecule" is intended to include nucleic acid molecules that contain one or more non-natural nucleotides, such as nucleotides having modifications to the base, the sugar, or the phosphate portion, or having one or more non-natural linkages, such as phosphothioate linkages. Such modifications can be advantageous in increasing the stability of the nucleic acid molecule, particularly when used in hybridization applications.

Furthermore, the term "nucleic acid molecule" is intended to include nucleic acid molecules modified to contain a detectable moiety, such as a radiolabel, a fluorochrome, a ferromagnetic substance, a luminescent tag or a detectable binding agent such as biotin. Nucleic acid molecules containing such moieties are useful as probes for detecting the presence or expression of a 2,5-DKG permease nucleic acid molecule.

In one embodiment, the isolated nucleic acid molecule encoding a polypeptide which has 2,5-DKG permease activity contains a nucleotide sequence having at least 80% identity to any of the 2,5-DKG permease nucleic acid molecules designated SEQ ID NOS:1, 3, 5, 7, or 9. Preferably, such a molecule will have at least 90% identity to any of these recited SEQ ID NOS, such as 95%, 98%, 99% or greater identity to SEQ ID NOS:1, 3, 5, 7, or 9.

In another embodiment, the isolated nucleic acid molecule encoding a polypeptide which has 2,5-DKG permease activity contains a nucleotide sequence which encodes a polypeptide having at least 80% identity to any of the 2,5-DKG permease polypeptides designated SEQ ID NOS:2, 4, 6, 8, or 10. Preferably, the encoded polypeptide will have at least 90% identity to any of 95%, 98%, 99% or greater identity to SEQ ID NOS:2, 4, 6, 8, or 10.

The term "percent identity" with respect to a nucleic acid molecule or polypeptide of the invention is intended to refer to the number of identical nucleotide or amino acid residues between the aligned portions of two sequences, expressed as a percent of the total number of aligned residues, as determined by comparing the entire sequences using a CLUSTAL V computer alignment and default parameters. CLUSTAL V alignments are described in Higgens, Methods Mol. Biol. 25:307-318 (1994), and an exemplary CLUSTAL V alignment of 2,5-DKG permease amino acid sequences is presented in Figure 1.

Due to the degeneracy of the genetic code, the nucleotide sequence of a native nucleic acid molecule can be modified and still encode an identical or substantially similar polypeptide. Thus, degenerate variants of SEQ ID NOS:1, 3, 5, 7, or 9 are exemplary invention nucleic acid molecules encoding polypeptides having 2,5-DKG permease activity.

Additionally, nucleic acid molecules encoding 2,5-DKG permeases from other species of microorganisms are exemplary invention nucleic acid molecules. The six permeases designated YiaX2, PE1, PE6, prmA, prmB and PK1, which were isolated from at least three, and likely four, different species of microorganisms, share substantial nucleotide sequence identity (YiaX2 is included for comparative purposes only). For example, the two most similar of the disclosed 2,5-DKG permease nucleotide sequences, SEQ ID NO:5 (PK1) and SEQ ID NO:1 (PE1), share 86% identity across their length. The two most dissimilar of the disclosed 2,5-DKG permease nucleotide sequences, SEQ ID NO:7 (prmA) and SEQ ID NO:9 (prmB), share 51% identity across their length. In contrast, a search of GenBank reveals no other nucleotide sequences, including sequences which encode transporter proteins and other transmembrane proteins, that exhibit significant identity or similarity to any of the disclosed 2,5-DKG permease nucleotide sequences over the entire length of their sequences.

The six permeases disclosed herein also share substantial amino acid sequence identity over their entire length, as described previously. For example, PK1 from *Klebsiella oxytoca* (SEQ ID NO:6), and PE1, from an environmental source (SEQ ID NO:2), are 93% identical at the amino acid level. The amino acid sequence in the GenBank database most closely related to a 2,5-DKG permease, which is a putative tartrate transporter from *Agrobacterium* vitis (GenBank Accession U32375 or U25634) is 33% identical to comparative SEQ ID NO:12 (YiaX2), and shares less identity with the other disclosed 2,5-DKG permeases. Other sequences with some degree of identity in the GenBank database to the disclosed 2,5-DKG permease include membrane transporter proteins from a variety of species, including phthalate transporter proteins from B. cepacia (AF152094) and *P. putida* (D13229); hydroxyphenylacetate transporters from *S.* dublin (AF144422) and *E. coli* (Z37980); and probable transporter proteins from *S. coelicolor* (AL136503 and AL132991) each of which has about 27% or less identity at the amino acid level to the recited SEQ ID NOS.

In view of the high degree of identity between different 2,5-DKG permease nucleic acid molecules and encoded polypeptides within a single species and between different microbial species, additional 2,5-DKG permeases from other species can be readily identified and tested. Thus, nucleic acid molecules as defined in the claims include nucleic acid molecules that encode polypeptides having 2,5-DKG permease activity from any microbial species. Microorganisms that contain 2,5-DKG permeases can be recognized by their ability to actively transport 2,5-DKG, such that they can grow on 2,5-DKG as the sole carbon source, or incorporate 2,5-DKG in an uptake assay. Such microorganisms can include, for example, bacteria, including Archaebacteria, gram positive and gram negative bacteria; yeast; and fungi.

Exemplary bacteria which contain 2,5-DKG permeases include Proteobacteria, and more specifically Enterobacteria and Pseudomonads (e.g. P. aeruginosa), as described in the Example. Exemplary Enterobacteria include species from the genera *Klebsiella* (e.g. *K. oxytoca,* from which SEQ ID NOS:5 and 11 were obtained) and Pantoea (e.g. *P. citrea*, from which SEQ ID NOS:7 and 9 were obtained, and *P. agglomerans*). Sources of such microorganisms include public repositories, such as the American Type Culture Collection (ATCC), and commercial sources. It will be appreciated that the taxonomy and nomenclature of bacterial genera are such that the same or similar strains are sometimes reported in the literature as having different names. For example, *Klebsiella oxytoca* (e.g. ATCC 13182) has alternatively been described as Aerobacter *aerogenes*, *Klebsiella aerogenes* and *Klebsiella pneumoniae.* Likewise, *Pantoea agglomerans* (e.g. ATCC 21998) has alternatively been described as *Erwinia herbicola* and *Acetomonas albosesamae.* The terms *"Klebsiella"* and "Pantoea," as used herein, are intended to refer to the genera of the strains deposited as ATCC 13182 and 21998, respectively.

Additionally, microorganisms from which 2,5-DKG permease nucleic acid molecules can be obtained are microorganisms present in environmental samples. For example, the 2,5-DKG permease nucleic acid molecules designated SEQ ID NOS:1 and 3 were obtained from environmental samples. As used herein, the term "environmental sample" refers to a sample obtained from natural or man-made environments, which generally contains a mixture of microorganisms.

Exemplary environmental samples are samples of soil, sand, freshwater or freshwater sediments, marine water or marine water sediments, industrial effluents, hot springs, thermal vents, and the like. Within an environmental sample there are likely to be microorganisms that are unidentified, and also microorganisms that are uncultivable. Isolation of 2,5-DKG permease molecules as defined in the claims from microorganisms present in environmental samples does not require either identification or culturing of the microorganism.

Furthermore, nucleic acid molecules of the invention include nucleic acid molecules encoding amino acid sequences that are modified by one or more amino acid additions, deletions or substitutions with respect to the native sequence of SEQ ID NOS:2, 4, 6, 8, or 10. Such modifications can be advantageous, for example, in enhancing the stability, expression level, enzymatic activity, or 2,5-DKG selectivity of the permease. If desired, such modifications can be randomly generated, such as by chemical mutagenesis, or directed, such as by site-directed mutagenesis of a native permease sequence, using methods well known in the art.

An amino acid sequence that is modified from a native permease amino acid sequence can include one or more conservative amino acid substitutions, such as substitution of an apolar amino acid with another apolar amino acid (such as replacement of leucine with an isoleucine, valine, alanine, proline, tryptophan, phenylalanine or methionine); substitution of a charged amino acid with a similarly charged amino acid (such as replacement of a glutamic acid with an aspartic acid, or replacement of an arginine with a lysine or histidine); or substitution of an uncharged polar amino acid with another uncharged polar amino acid (such as replacement of a serine with a glycine, threonine, tyrosine, cysteine, asparagine or glutamine). A modified amino acid sequence can also include one or more nonconservative substitutions without adversely affecting the desired biological activity.

Computer programs known in the art can provide guidance in determining which amino acid residues can be substituted without abolishing the enzymatic activity of a 2,5-DKG permease (see, for example, Eroshkin et al., Comput. Appl. Biosci. 9:491-497 (1993)).

Additionally, guidance in modifying amino acid sequences while retaining or enhancing functional activity is provided by aligning homologous 2,5-DKG permease polypeptides from various species (see Figure 1). It is well known in the art that evolutionarily conserved amino acid residues and domains are more likely to be important for maintaining biological activity than less well-conserved residues and domains. Thus, it would be expected that substituting a residue which is highly conserved among the six 2,5-DKG permeases shown in Figure 1 (or among the members of the two structural families of permeases, defined as SEQ ID NOS:2, 6 and 10, and SEQ ID NOS:4, 8 and 12) (SEQ ID NO.12 is included for comparative purposes only) with a non-conserved residue may be deleterious, whereas making the same substitution at a residue which varies widely among the different permeases would likely not have a significant effect on biological activity.

A comparison of the amino acid sequences of PE1 (SEQ ID NO:2), which transports both 2,5-DKG and 2-KLG, and PK1 (SEQ ID NO:6), which selectively transports 2,5-DKG, indicates that the regions responsible for 2,5-DKG selectivity must reside in the 7% of amino acids which differ between these two sequences. Therefore, modifying all or some of these differing residues in a 2,5-DKG permease to those found in the PK1 sequence would be expected to increase 2,5-DKG selectivity of the permease.

Alignment of the six 2,5-DKG permeases described herein also provides guidance as to regions where additions and deletions are likely to be tolerated. For example the N and C termini, and the region around amino acids 225-250 (based on the numbering of comparative SEQ ID NO:12 (yiaX2)) appear to be regions that are relatively tolerant of amino acid insertions and deletions, as evidenced by gaps in the sequence alignment. Modified 2,5-DKG permeases can thus include "tag" sequences at such sites, such as epitope tags, histidine tags, glutathione-S-transferase (GST) and the like, or sorting sequences. Such additional sequences can be used, for example, to facilitate purification or characterization of a recombinant 2,5-DKG permease.

It will be appreciated that confirmation that any particular nucleic acid molecule is a nucleic acid molecule of the invention can be obtained by determining the 2,5-DKG permease activity of the encoded polypeptide, using one or more of the functional assays described herein.

The invention further provides an isolated nucleic acid molecules as defined in the claims encoding a polypeptide which has 2,5-DKG permease activity, wherein the nucleic acid molecule is operatively linked to a promoter of gene expression. The term "operatively linked," as used herein, is intended to mean that the nucleic acid molecule is positioned with respect to either the endogenous promoter, or a heterologous promoter, in such a manner that the promoter will direct the transcription of RNA using the nucleic acid molecule as a template.

Methods for operatively linking a nucleic acid to a desired promoter are well known in the art and include, for example, cloning the nucleic acid into a vector containing the desired promoter, or appending the promoter to a nucleic acid sequence using PCR. A nucleic acid molecule operatively linked to a promoter of RNA transcription can be used to express 2,5-DKG transcripts and polypeptides in a desired host cell or in vitro transcription-translation system.

The choice of promoter to operatively link to an invention nucleic acid molecule will depend on the intended application, and can be determined by those skilled in the art. For example, if a particular gene product may be detrimental to a particular host cell, it may be desirable to link the invention nucleic acid molecule to a regulated promoter, such that gene expression can be turned on or off. An exemplary inducible promoter known in the art is the lacPO promoter/operator, which is repressed by the *lacI^{q}* gene product provided by certain host cells, and induced in the presence of 0.01 to 1 mM IPTG (see Example, below). For other applications, weak or strong constitutive promoters may be preferred.

The invention further provides a vector containing an isolated nucleic acid molecule as defined in the claims encoding a polypeptide which has 2,5-DKG permease activity. The vectors of the invention will generally contain elements such as a bacterial origin of replication, one or more selectable markers, and one or more multiple cloning sites. The choice of particular elements to include in a vector will depend on factors such as the intended host cell or cells; whether expression of the inserted sequence is desired; the desired copy number of the vector; the desired selection system, and the like. The factors involved in ensuring compatibility between a host and a vector for different applications are well known in the art.

In applications in which the vectors will be used for recombinant expression of the encoded polypeptide, the isolated nucleic acid molecules will generally be operatively linked to a promoter of gene expression, as described above, which may be present in the vector or in the inserted nucleic acid molecule. In cloning and subcloning applications, however, promoter elements need not be present.

An exemplary vector suitable for both cloning applications and for expressing 2,5-DKG permeases in different bacterial species is the low copy number plasmid pCL1920 described by Lerner et al., Nucleic Acids Res. 18:4621 (1994), which contains a spectinomycin resistance gene (see Example, below).

Also provided are cells containing an isolated nucleic acid molecule as defined in the claims encoding a polypeptide which has 2,5-DKG permease activity. The isolated nucleic acid molecule will generally be contained within a vector compatible with replication in the particular host cell. However, for certain applications, incorporation of the nucleic acid molecule into the bacterial genome will be preferable.

The cells of the invention can be any cells in which a 2,5-DKG permease will be expressed and folded into an active conformation. Guidance in choosing appropriate host cells is provided by identifying cell types which express other functional 10 to 12 transmembrane transporter proteins. For example, 10 to 12 transmembrane transporter proteins are found in a variety of bacterial species, as well as in yeast (e.g. *S. pombe*)*, Arabidopsis,* and *Drosophila.* Therefore, depending on the particular application for the host cell, a host cell of the invention can be a bacterial cell, yeast, Arabidopsis or Drosophila cell.

In a preferred embodiment, the cell is a bacterial cell. The choice of bacterial cell will depend on the intended application. For example, for routine subcloning applications, the cell can be any convenient laboratory strain of bacteria, such as *E. coli,* which can be transformed with the isolated nucleic acid molecules and vectors of the invention by methods well known in the art.

For assessment of encoded 2,5-DKG permease activity, the cell can be a bacterial strain suitable for metabolic assays, such as a strain which endogenously expresses, or which is engineered to express, enzymes that catalyze the conversion of 2,5-DKG to essential products. An exemplary strain suitable for metabolic assays is the *K. oxytoca* Δ*yiaX2* [*tkr idnO*] strain designated MGK002[pDF33] described further in the Example, below, which provides for the conversion of intracellular 2,5-DKG to gluconic acid, which can be used as a carbon and energy source.

For use in the commercial bioproduction of 2,5-DKG metabolites, the cell can be a bacterial strain which endogenously expresses, or which is engineered to express, a 2,5-DKG reductase. As described in U.S. Patent No. 5,032,514, 2,5-DKG reductases are found in genera including Brevibacterium, Arthrobacter, Micrococcus, Staphylococcus, Pseudomonas, Bacillus, Citrobacter and Corynebacterium. Therefore, a cell of the invention can be a bacterial cell of any of these genera, or a bacterial cell engineered to express a 2,5-DKG reductase of any of these genera.

A cell able to produce 2,5-DKG metabolites will preferably also be able to catalyze the extracellular production of 2,5-DKG from an inexpensive carbon source, such as glucose. An exemplary pathway from D-glucose to 2,5-DKG involves the enzymatic conversion of D-glucose to D-gluconic acid (catalyzed by D-glucose dehydrogenase), from D-gluconic acid to 2-Keto-D-gluconic acid (catalyzed by D-gluconate dehydrogenase), and from 2-Keto-D-gluconic acid to 2,5-DKG (catalyzed by 2-Keto-D-gluconic acid dehydrogenase), as is shown in Figure 2. These steps can be carried out by organisms of several genera, including Gluconobacter, Acetobacter and Erwinia (also called Pantoea).

A bacterial cell useful for the production of 2-KLG from D-glucose is the *Pantoea aggolmerans* (also referred to as *Erwinia herbicola* or *Acetomonas albosesamae*) strain described in U.S. Patent No. 5,032,514, designated ATCC 21998 ptrp 1-35 tkrAΔ3, or a derivative of this strain with improved properties. Contemplated improvements to this strain, which can be produced by genetic engineering, include deletion of enzymes that divert glucose to metabolites other than 2-KLG, such that yield of 2-KLG is increased. Other contemplated improvements to this strain include mutations that provide for improved recovery and purification of 2-KLG.

The *Pantoea* strain described in U.S. Patent No. 5,032,514 recombinantly expresses a 2,5-DKG reductase from Corynebacterium (described in U.S. Patent No. 4,757,012). The strain further contains a mutation that results in a non-functional tkrA gene and is thus deficient in 2-keto reductase activity. Mutation of the tkrA gene is advantageous in reducing metabolic diversion of 2-KLG to L-idonic acid, and metabolic diversion of 2,5-DKG to 5-keto-D-gluconate from 2-KLG.

Expression of one or more 2,5-DKG permeases of the invention in such cells significantly increases overall production of 2-KLG from D-glucose, which lowers the cost of commercial production of ascorbic acid.

The cells of the invention can contain one, two or more isolated nucleic acid molecules as defined by the claims that encode polypeptides having 2,5-DKG permease activity. For example, the cell can contain an isolated nucleic acid molecule encoding at least one polypeptide having at least 80% identity to any of SEQ ID NOS:2, 4, 6, 8, or 10, and optionally will contain two or more such nucleic acid molecules, in any combination. Preferably, at least one such encoded polypeptide selectively transports 2,5-DKG.

In a preferred embodiment, a bacterial cell of the invention suitable for bioproduction of 2-KLG contains an isolated nucleic acid molecule encoding a polypeptide having at least 95% identity to the 2,5-DKG selective permease designated SEQ ID NO:8 (prmA); and optionally further containing at least one isolated nucleic acid molecule encoding a polypeptide having at least 95% identity to a 2,5-DKG permease selected from the group consisting of SEQ ID NO:4 (PE6), SEQ ID NO:10 (prmB) and SEQ ID NO:6 (PK1).

The invention also provides a method of enhancing production of 2-KLG. The method consists of culturing a bacterial cell, wherein the cell contains an isolated nucleic acid molecule as defined by the claims encoding a polypeptide which has 2,5-DKG permease activity, under conditions wherein the encoded 2,5-DKG permease is expressed and intracellular 2,5-DKG is converted to 2-KLG. Cells suitable for this purpose, such as the Pantoea strain described in U.S. Patent No. 5,032,514, have been described above. Optionally, the 2-KLG so produced can be chemically or enzymatically converted to a desired product such as ascorbic acid, following methods known in the art.

For certain applications, such as for detecting 2,5-DKG expression in a cell or library, it will be desirable to use nucleic acid molecules as defined in the claims that specifically hybridize to a nucleic acid molecule encoding a 2,5-DKG permease. The term "specifically hybridize" refers to the ability of a nucleic acid molecule to hybridize, under stringent hybridization conditions as described below, to a nucleic acid molecule that encodes a 2,5-DKG permease, without hybridizing to a substantial extent under the same conditions with nucleic acid molecules that do not encode 2,5-DKG permeases, such as unrelated molecules that fortuitously contain short regions of identity with a permease sequence. Thus, a nucleic acid molecule that "specifically hybridizes" is of a sufficient length and contains sufficient distinguishing sequence from a 2,5-DKG permease to be characteristic of the 2,5-DKG permease. Such a molecule will generally hybridize, under stringent conditions, as a single band on a Northern blot or Southern blot prepared from mRNA of a single species.

As used herein, the term "stringent conditions" refers to conditions equivalent to hybridization of a filter-bound nucleic acid molecule to a nucleic acid in a solution containing 50% formamide, 5X Denhart's solution, 5X SSPE, 0.2% SDS at 42°C, followed by washing the filter in 0.1X SSPE, and 0.1% SDS at 65°C twice for 30 minutes. Equivalent conditions to the stringent conditions set forth above are well known in the art, and are described, for example in Sambrook et al., Molecular Cloning: A Laboratory Manual, Cold Spring Harbor Laboratory, New York (1992).

Nucleotide sequences that are characteristic of each of SEQ ID NOS:1, 3, 5, 7 or 9, or which are common to two, three or more of SEQ ID NOS:1, 3, 5, 7, 9 or 11 can readily be determined by aligning the sequences using a CLUSTAL V alignment program. Oligonucleotides containing regions which are common to two or more different 2,5-DKG permease nucleic acid molecules can advantageously be used as PCR primers or hybridization probes to isolate or detect nucleic acid molecules encoding 2,5-DKG permeases from other species.

Isolated nucleic acid molecules as defined in the claims which encode polypeptides having 2,5-DKG permease activity, will be subsequently referred as "2,5-DKG permease nucleic acid molecules."

The isolated 2,5-DKG permease nucleic acid molecules as defined in the claims can be prepared by methods known in the art. The method chosen will depend on factors such as the type and size of nucleic acid molecule one intends to isolate; whether or not it encodes a biologically active polypeptide (e.g. a polypeptide having permease activity); and the source of the nucleic acid molecule. Those skilled in the art can isolate or prepare 2,5-DKG permease nucleic acid molecules as genomic DNA or desired fragments therefrom; as full-length cDNA or desired fragments therefrom; or as full-length mRNA or desired fragments therefrom, from any microorganism of interest.

An exemplary method of preparing a 2,5-DKG permease nucleic acid molecule is by isolating a recombinant construct which encodes and expresses a polypeptide having 2,5-DKG permease activity. As described in the Example, one useful method is to provide a metabolic selection system where bacterial cell growth is made dependent on expression of a 2,5-DKG permease, introducing expressible DNA, such as a cDNA or genomic library, into the assay cells, selecting surviving cells under the selective conditions, and isolating the introduced DNA. Alternatively, a screening method can be designed, such that a cell will exhibit a detectable signal only when expressing a functional 2,5-DKG permease. An exemplary detectable signal is intracellular incorporation of a detectable label present on 2,5-DKG. Additionaly screening and selection strategies suitable for identifying nucleic acid molecules encoding metabolic enzymes are described, for example, in PCT publication WO 00/22170 and U.S. Patent Nos. 5,958,672 and 5,783,431.

A further method for producing an isolated 2,5-DKG permease nucleic acid molecule involves amplification of the nucleic acid molecule using 2,5-DKG permease-specific primers and the polymerase chain reaction (PCR). Using PCR, a 2,5-DKG permease nucleic acid molecule having any desired boundaries can be amplified exponentially starting from as little as a single gene or mRNA copy, from any cell having a 2,5-DKG permease gene.

Given the high degree of identity among the six disclosed 2,5-DKG permeases, those skilled in the art can design suitable primers for isolating additional 2,5-DKG permease nucleic acid molecules. Such primers are preferably degenerate oligonucleotides that encode, or are complementary to, short consensus amino acid sequences present in two or more of the 2,5-DKG permeases disclosed herein, such as oligonucleotides that encode 10 or more contiguous amino acids present in at least two of SEQ ID NOS: 2, 6 and 10, or oligonucleotides that encode 10 or more contiguous amino acids present in at least two of SEQ ID NOS:4, 8 and 12 (SEQ ID NO. 12 is included for comparative purposes only). Such sequences can be determined from an alignment of amino acid sequences shown in Figure 1. Exemplary amino acid sequences present in at least two of SEQ ID NOS:2, 6 and 10 are amino acids 19-31, 115-124, 146-156, and 339-348 of SEQ ID NO:2. Exemplary amino acid sequences present in at least two of SEQ ID NOS:4, 8 and 12 are amino acids 55-64, 60-69, 252-261, and 370-379 of SEQ ID NO:8.

Methods are well known in the art to determine or modify PCR reaction conditions when using degenerate primers to isolate a desired nucleic acid molecule. The amplified product can subsequently be sequenced, used as a hybridization probe, or used for 5' or 3' RACE to isolate flanking sequences, following procedures well known in the art and described, for example, in Ausubel et al., Current Protocols in Molecular Biology, John Wiley & Sons, New York (2000).

Given the high degree of sequence identity and structural relatedness among the six disclosed 2,5-DKG permeases, homologs from any other species can readily be identified by either hybridization or antibody screening. For example, an isolated 2,5-DKG permease nucleic acid molecule can be identified by screening a library, such as a genomic library, cDNA library or expression library, with a detectable nucleic acid molecule or antibody. Such libraries are commercially available from a variety of microorganisms, or can be produced from any available microorganism or environmental sample of interest using methods described, for example, in PCT publication WO 00/22170. The library clones identified as containing 2,5-DKG permease nucleic acid molecules can be isolated, subcloned and sequenced by routine methods.

Furthermore, 2,5-DKG permease nucleic acid molecules can be produced by direct synthetic methods. For example, a single stranded nucleic acid molecule can be chemically synthesized in one piece, or in several pieces, by automated synthesis methods known in the art. The complementary strand can likewise be synthesized in one or more pieces, and a double-stranded molecule made by annealing the complementary strands. Direct synthesis is particularly advantageous for producing relatively short molecules, such as oligonucleotide probes and primers, and also for producing nucleic acid molecules containing modified nucleotides or linkages.

The invention also provides an isolated polypeptide as defined in the claims which has 2,5-DKG permease activity. Such isolated polypeptides, when expressed in their normal configuration at the cell membrane, are useful in applications in which enhanced uptake of 2,5-DKG is desirable, such as in bioproduction of 2-KLG. The isolated polypeptides of the invention can also be added to a culture medium, preferably in a membrane vesicle, to compete with membrane-bound permeases for 2,5-DKG, and thus to stop 2,5-DKG uptake. Thus, isolated polypeptides having 2,5-DKG permease activity can be used to regulate production of 2,5-DKG metabolites.

An "isolated" polypeptide of the invention is altered by the hand of man from how it is found in its natural environment. For example, an isolated 2,5-DKG permease can be a molecule that is recombinantly expressed, such that it is present at a higher level in its native host, or is present in a different host. Alternatively, an "isolated" 2,5-DKG permease of the invention can be a substantially purified molecule. Substantially purified 2,5-DKG permeases can be prepared by methods known in the art. Specifically with respect to a polypeptide encoding the *yiaX2* polypeptide designated SEQ ID NO:12 (which is included for comparative purposes only), the term "isolated" is intended to mean that polypeptide is not present in association with the polypeptides expressed by other genes in the K. *oxytoca yia* operon, such as the genes designated *lyxK* and *orf1*, described in WO 00/22170.

In one embodiment, an isolated polypeptide having 2,5-DKG permease activity contains an amino acid sequence having at least 80% identity to an amino acid sequence selected from the group consisting of SEQ ID NOS:2, 4, 6, 8, or 10. Preferably, the encoded polypeptide will have at least 90% identity to any of the recited SEQ ID NOS, including 95%, 98%, 99% or greater identity.

For the production of antibodies that recognize 2,5-DKG permeases as defined in the claims, the 2,5-DKG polypeptide need not be in its native configuration to be recognized.

The structural and functional characteristics and applications of 2,5-DKG permease polypeptides of the invention have been described above with respect to the encoding nucleic acid molecules as defined in the claims, and are equally applicable in reference to the isolated polypeptides as defined in the claims. Isolated polypeptides having 2,5-DKG permease activity, will subsequently be referred to as "2,5-DKG permeases."

Methods for recombinantly producing 2,5-DKG permeases have been described above with respect to nucleic acid molecules, vectors and cells of the invention. 2,5-DKG permeases can alternatively be prepared by biochemical procedures, by isolating membranes from bacteria that naturally express, or recombinantly express, 2,5-DKG permeases. The membranes can be further fractionated by size or affinity chromatography, electrophoresis, or immunoaffinity procedures, to achieve the desired degree of purity. Purification can be monitored by a variety of procedures, such as by immunoreactivity with 2,5-DKG permease antibodies, or by a functional assay.

Alternatively, 2,5-DKG permeases can be produced by chemical synthesis. If desired, such as to optimize their functional activity, stability or bioavailability, such chemically synthesized molecules can include D-stereoisomers, non-naturally occurring amino acids, and amino acid analogs and mimetics. Sawyer, Peptide Based Drug Design, ACS, Washington (1995) and Gross and Meienhofer, The Peptides· Analysis, Synthesis, Biology, Academic Press, Inc., New York (1983). For certain applications, such as for detecting the polypeptide, it can also be useful to incorporate one or more detectably labeled amino acids into a chemically synthesized permease, such as radiolabeled or fluorescently labeled amino acids.

An isolated 2,5-DKG permease as defined in the claims can further be conjugated to carrier molecules, such as keyhole lympet hemocyanin, which can enhance recognition by the immune system of the isolated 2,5-DKG permease for production of antibodies. For certain applications, such as to increase the stability or bioactivity of the molecule, or to facilitate its identification, the 2,5-DKG permease can be chemically or enzymatically derivatized, such as by acylation, phosphorylation or glycosylation.

The invention also provides an antibody specific for a polypeptide having 2,5-DKG permease activity, such as an antibody specific for a polypeptide having the amino acid sequence of any of SEQ ID NOS:2, 4, 6, 8, or 10. The antibodies of the invention can be used, for example, to detect or isolate 2,5-DKG permeases from expression libraries or cells.

The term "antibody," as used herein, is intended to include molecules having specific binding activity for a 2,5-DKG permease as defined in the claims of at least about 1 x 10⁵ M⁻¹, preferably at least 1 x 10⁷ M⁻¹, more preferably at least 1 x 10⁹ M⁻¹. The term "antibody" includes both polyclonal and monoclonal antibodies, as well as antigen binding fragments of such antibodies (e.g. Fab, F(ab')₂, Fd and Fv fragments and the like). In addition, the term "antibody" is intended to encompass non-naturally occurring antibodies, including, for example, single chain antibodies, chimeric antibodies, bifunctional antibodies, CDR-grafted antibodies and humanized antibodies, as well as antigen-binding fragments thereof.

Methods of preparing and isolating antibodies, including polyclonal and monoclonal antibodies, using peptide and polypeptide immunogens, are well known to those skilled in the art and are described, for example, in Harlow and Lane, Antibodies: A Laboratory Manual, Cold Spring Harbor Laboratory Press (1988). Non-naturally occurring antibodies can be constructed using solid phase peptide synthesis, can be produced recombinantly or can be obtained, for example, by screening combinatorial libraries consisting of variable heavy chains and variable light chains. Such methods are described, for example, in Huse et al. Science 246:1275-1281 (1989); Winter and Harris, Immunol. Today 14:243-246 (1993); Ward et al., Nature 341:544-546 (1989); Hilyard et al., Protein Engineering: A practical approach (IRL Press 1992); and Borrabeck, Antibody Engineering, 2d ed. (Oxford University Press 1995).

The following example is intended to illustrate but not limit the present invention.

### EXAMPLE

This example shows the isolation and characterization of nucleic acid molecules encoding six novel polypeptides having 2,5-DKG permease activity.

### Identification of yiaX2 as a 2,5-DKG permease (this example is included for comparative purposes only)

WO 002170 describes the identification and sequencing of an operon from *Klebsiella oxytoca,* designated the *yia* operon, which contains eight putative open reading frames. Because disruption of this operon abolished the ability of *K. oxytoca* to utilize ascorbic acid as the sole carbon source, the *yia* operon was predicted to be involved in the catabolism of ascorbic acid. The functions of the polypeptides encoded by the individual open reading frames in the yia operon were not described in WO 002170.

It was determined that *K. oxytoca* was able to grow on 2,5-DKG as a sole carbon source and, therefore, it was concluded that *K. oxytoca* expressed a 2,5-DKG permease. It was predicted that such a permease would share structural properties with known bacterial transporter proteins, such as multiple transmembrane segments. One of the uncharacterized open reading frames in the yia operon, designated *yiaX2*, encoded a transmembrane polypeptide with about 33% identity to a known tartrate transporter, and was thus considered a candidate 2,5-DKG permease.

In order to determine whether *yiaX2* encoded a 2,5-DKG permease, this gene was deleted from the chromosome of the *K*. *oxytoca* strain designated M5a1. M5a1 has also been described in the literature as *K. pneumonia* (see, for example, Streicher et al., Proc. Natl. Acad. Sci. 68:1174-1177 (1971)). The *yiaX2* deletion mutant was constructed by joining sequences immediately upstream and downstream of the *yiaX2* gene in a three-way ligation with the pMAK705 integration vector (described in Hamilton et al., J. Bacteriol. 171:4617-4622 (1989)). A fragment of about 1 kb in the *orf1* gene was amplified using oligonucleotides 5'-ACCCAAGCTTCACCAAAAGAGTGAAGAGGAAG-3' (SEQ ID NO:17) and 5'-CGTATCTAGAAAAATATTCTGGTGATGAAGGTGA-3 (SEQ ID NO:18), and digested with HindIII and XbaI. A fragment of a similar size in the *lyxK* gene was amplified with oligonucleotides 5'-AGACTCTAGATCCACATAAACGCACTGCGTAAAC-3' (SEQ ID NO:19) and 5'-GAGGGGATCCTGGCTTCGTGAACGATATACTGG-3' (SEQ ID NO:20), and digested with XbaI and BamHI. The two resulting fragments were ligated together between the HindIII and BamHI sites of the vector pMAK705. The resulting plasmid was transformed into *K*. *oxytoca* strain M5a1, and candidates in which the deletion construct had integrated by double crossover were obtained as described in Hamilton et al., supra (1989). The designation of the resulting *K. oxytoca ΔyiaX2* strain is MGK002. The *yiaX2*-deficient phenotype was verified by by PCR analysis.

As described below, the *K. oxytoca ΔyiaX2* [*tkr idnO*] strain was determined to grow very inefficiently on 2,5-DKG as the sole carbon source, and not to grow on 2-KLG. Confirmation that *yiaX2* encoded a polypeptide having 2,5-DKG and 2-KLG permease activities was obtained by determining that adding back the gene restored the ability of the *K. oxytoca ΔyiaX2* [*tkr idnO*] to grow well on either 2,5-DKG or 2-KLG (see below).

### Construction of K. oxytoca ΔyiaX2 [tkr idnO]

In order to identify additional 2,5-DKG permeases, and preferably permeases selective for 2,5-DKG, a metabolic selection strategy was utilized. As described in WO 00/22170, metabolic selection is advantageous in allowing rapid identification of functional genes from uncharacterized and even unculturable microorganisms, without any prior sequence information.

A tester strain for the metabolic selection of nucleic acid molecules encoding 2,5-DKG permeases was prepared by engineering *K. oxytoca ΔyiaX2* to express enzymes involved in the catabolism of 2,5-DKG to gluconic acid, which can be converted to carbon and energy. Enzymes capable of catabolizing 2,5-DKG to gluconic acid are encoded by the tkr and *idnO* genes of the *tkr idnD idnO* operon designated SEQ ID NO:13.

The *tkr idnD idnO* operon (SEQ ID NO:13) was subcloned into the high copy number vector pUC19 and the resulting clone, designated pDF33, was transformed into *K. oxytoca* Δ*yiaX2*. The resultingtester strain (designated MGK002[pDF33] or *K. oxytoca ΔyiaX2* [*tkr idnO*]) thus expresses all polypeptides required for the utilization of 2,5-DKG as a sole carbon source, but is deficient in 2,5-DKG permease activity to transport extracellular 2,5-DKG into the cell. Therefore, a nucleic acid molecule that encodes a 2,5-DKG permease, upon expression in the tester strain, should confer the ability of the tester strain to grow on 2,5-DKG. The metabolic selection strategy is shown schematically in Figure 3.

To validate the proposed metabolic selection strategy, as a positive control the *yiaX2* gene was reintroduced into the tester strain to confirm that it conferred the ability to grow on 2,5-DKG and 2-KLG. The yiaX2 open reading frame (nucleotides 3777 to 5278 of SEQ ID NO:19 of WO 00/22170) was PCR-amplified using olignucleotides 5'-AATAGGATCCTTCATCACCAGAATATTTTTA-3' (SEQ ID NO:21) and 5'-CATAGGTACCGGCTTTCAGATAGGTGCC-3' (SEQ ID NO:22) digested with BamH1 and Kpn1 and ligated into pCL1920 (Lerner et al., Nucl. Acids. Res. 18:4631 (1990); and see description below) previously digested with the same restriction enzymes. *K. oxytoca* Δ*yiaX2* [*tkr idnO*], transformed with the resulting construct, was able to grow overnight at 30°C on M9 minimal agar medium supplemented with either 2-KLG or 2,5-DKG (0.25%) and 0.1 mM IPTG. Therefore, *K. oxytoca ΔyiaX2* [*tkr idnO*] was confirmed to be an appropriate tester strain to identify additional novel 2,5-DKG permeases, and to determine their selectivity.

### Construction of bacterial genomic libraries

The cloning vector used for constructing the above positive control and for preparing bacterial genomic libraries is plasmid pCL1920 (Lerner et al., supra, 1990), a low-copy number expression vector which carries a spectinomycin/streptomycin resistance determinant. Expression is driven by the lacPO promoter/operator region which is repressed by the *lacI^{q}* gene product when provided by the host, and induced in the presence of 0.01 to 1mM IPTG.

Genomic DNA from the following species and isolates was prepared according to the method outlined below: Pantoea citrea (ATCC 39140), *Klebsiella oxytoca* MGK002 (Δ*yiaX2*)*, Pseudomonas aeruginosa,* and a mixture of 25 environmental isolates, obtained from 18 different soil and water samples, and able to grow on 2,5-DKG as the sole carbon source. *Klebsiella oxytoca* MGK002 (Δ*yiaX2*) was among the bacteria chosen because there was a slight amount of background growth observable in the tester strain on 2,5-DKG as the sole carbon source, and some 2,5-DKG permease activity in an uptake assay. However, the tester strain did not grow on 2-KLG, and exhibited no detectable 2-KLG uptake, suggesting the presence of a second 2,5-DKG permease with selectivity for 2,5-DKG in *K. oxytoca*.

Five milliliters of an overnight culture in LB (30°C) were centrifuged for 5 min at 6,000 rpm. Pellets were washed with 1.5 ml Tris 10mM, EDTA 1mM pH 8.0 (TE), centrifuged again and resuspended in 0.4 ml TE. Lysozyme (5mg/ml) and RNase (100pg/ml) were added and cells were incubated for 10 min at 37°C. Sodium dodecylsulfate (SDS) was added to a final concentration of 1% and the tubes were gently shaken until lysis was complete. One hundred microliters of a 5N NaClO₄ stock solution were added to the lysate. The mixture was extracted once with one volume of phenol:chloroform (1:1) and once with one volume of chloroform. Chromosomal DNA was precipitated by adding 2 ml of cold (-20°C) ethanol and gently coiling the precipitate around a curved Pasteur pipette. DNA was dried for 30 min at room temperature and resuspended in 50 to 100 µl of Tris 10mM, EDTA 1mM, NaCl 50mM pH 8.0 to obtain a DNA concentration of 0.5 to 1 µg/µl. Genomic DNA preparations from each environmental isolate were mixed in equal ratios to prepare a single mixed library.

For each preparation, an aliquot of 10-15 µl of genomic DNA was subjected to Sau3A controlled digestion in order to obtain fragments ranging between 3 to 20kb in size. Half that amount was ligated with the low-copy number expression vector pCL1920, which had previously been digested with BamHI and dephosphorylated. The resulting genomic libraries were transformed into *E.coli* DH10B electrocompetent cells (GIBCO-BRL) and briefly amplified overnight at 30°C on LB-agar supplemented with 100µg/ml spectinomycin. For each library, 30,000 to 120,000 clones were plated out and plasmid DNA was bulk-extracted using standard procedures. Insert size was randomly checked and the amplified libraries were stored in the form of plasmid DNA at -20°C for further use in the tester strain.

### Selection, identification and sequencing of permease genes

An aliquot of each genomic library was introduced by electroporation into the *K. oxytoca* Δ*yiaX2* [*tkr idnO*] (MGK002 [pDF33]) strain. The amount of DNA used in the transformation was adjusted in order to plate out 5 x 10⁵ to 1 x 10⁶ clones per library on the selective medium. Each selection round was plated on LB-agar containing 100µg/ml spectinomycin, then replica-plated onto M9-agar plates containing 2.5% 2,5-DKG and 0.1 mM IPTG and adjusted to pH 4.5. The clones that grew on 2,5-DKG⁻ were transferred into K. *oxytoca* Δ*yiaX2* (MGK002) devoid of plasmid pDF33, to verify that the *tkr idnD0* pathway was indeed required for growth of those clones on 2,5-DKG. A brief genetic characterization was performed to eliminate identical clones. Following preliminary 2,5-DKG/ 2-KLG uptake assays, 5 clones were retained for further analysis: 2 originated from the Pantoea citrea library, 1 from K. *oxytoca* and 2 from the mixed environmental library.

In all cases, DNA sequencing of the vector inserts revealed the presence of a nucleotide sequence (SEQ ID NOS:1, 3, 5, 7 and 9) encoding a polypeptide (SEQ ID NOS:2, 4, 6, 8 and 10) displaying homology with published transporters and with *yiaX2* (SEQ ID NO:11, and its encoded polypeptide SEQ ID NO:12). Also present on these inserts were other orfs, and in most cases an endogenous promoter.

The insert containing both of the prmA and prmB orfs (SEQ ID NOS:7 and 9) was about 9kb, and also contained an orf homologous to bacterial *idnO*, two orfs encoding transcriptional repressors, an orf of unknown function, and 3 orfs encoding homologs of *E. coli* polypeptides involved in nitrate utilization.

The insert containing the PE1 orf (SEQ ID NO:1) was about 3kb, and also contained a putative dehydro-deoxygluconokinase gene closely related to the B. *subtilis kdgK* gene and a homolog of the *E. coli ydcG* gene.

The insert containing the PE6 orf (SEQ ID NO:3) was about 6.7kb. The genomic environment of PE6 appeared similar to the *yia* operon of *E. coli* and *K*. *oxytoca,* as SEQ ID NO:4 was preceded by a *yiaL* homolog and a *yiaK* homolog was also present on the insert.

The insert containing the PK1 orf (SEQ ID NO:5) was about 5.5kb. In contrast to the other inserts, this insert did not appear to contain an endogenous promoter, indicating that the PK1 orf was apparently transcribed from the vector's promoter. The PK1 orf was directly followed by a tkr homolog.

Nucleic acid molecules encoding each 2,5-DKG permease were reintroduced into *K. oxytoca* Δ*yiaX2* [*tkr idnO*] and the resulting strains assayed for growth on 2,5-DKG and 2-KLG, and also assayed for uptake of radiolabeled 2,5-DKG and 2-KLG.

The uptake assays were performed by mixing radioactive 2-KLG or 2,5-DKG with IPTG-induced cells, removing aliquots at regular intervals, and measuring both the decrease in radioactivity in the supernatant and the appearance of radioactivity in the cells over time. The results of the growth assays and 2,5-DKG uptake assay are shown in Table 1, below.

**Table 1**

| **Recombinantly expressed on 2,5 DKG Permease** | **Cell Growth 2,5-DKG** | **Cell Growth on 2-KLG** | **2,5-DKG Uptake (g/l/h)** |
|---|---|---|---|
| YiaX2 | + | ++ | 3.7 |
| PE1 | ++ | ++ | 4.2 |
| PE6 | ++ | ++ | 5.0 |
| prmA | ++ | ++ | 5.5 |
| prmB | +/- | ND | 0.9 |
| prmA and prmB | ++ | ND | 9.9 |
| PK1 | ++ | _ | 4.2 |
| Control (*K. oxytoca ΔyiaX2*/ *tkr*/*idn0*) | bkgd | - | 1.0 |

Nucleic acid molecules encoding the different permeases were also subcloned into a variety of vectors, including the high copy number vector pSE380 (which contains a tac promoter), the medium copy number vector pACYC184 (which is promoterless), or the low copy number vector pCL1920, and introduced into a *Pantoea* strain suitable for bioproduction of 2-KLG from glucose (see U.S. Patent No. 5,032,514). The resulting strains were assessed under biofermentation conditions to determine which combinations of nucleic acid molecules, promoters and vectors are optimal for enhancing 2-KLG production.

## Claims

1. An isolated nucleic acid molecule encoding a polypeptide which has 2,5-DKG permease activity, said nucieic acid molecule comprising
(a) a nucleotide sequence having at least 80% identity to a nucleotide sequence selected from the group consisting of SEQ ID NOS:1, 3, 5, 7 and 9; or
(b) a nucleotide sequence selected from the group consisting of SEQ ID NOS:1, 3, 5, 7 and 9; or
(c) a nucleotide sequence which encodes a polypeptide having at least 80% identity to an amino acid sequence selected from the group consisting of SEQ ID NOS:2, 4, 6, 8 and 10.

2. The isolated nucleic acid molecule of claim 1, which encodes a polypeptide having an amino acid sequence selected from the group consisting of SEQ ID NOS:2, 4, 6, 8 and 10.

3. The isolated nucleic acid molecule of claim 1 operatively linked to a promoter of gene expression.

4. The isolated nucleic acid molecule of claim 3, wherein said promoter is a *lac* promoter.

5. A vector comprising the isolated nucleic acid molecule of claim 3 or claim 4.

6. The vector of claim 5, comprising a spectinomycin resistance gene.

7. A bacterial cell, comprising the vector of claim 5 or claim 6.

8. The bacterial cell of claim 7, wherein said isolated nucleic acid molecule comprises a nucleotide sequence which encodes a polypeptide which has 2,5-DKG permease activity, said polypeptide having an amino acid sequence at least 80% identical to an amino acid sequence selected from the group consisting of SEQ ID NO:2, 4, 6, 8 and 10.

9. The bacterial cell of claim 8, wherein said amino acid sequence is at least 95% identical to SEQ ID NO:8.

10. The bacterial cell of claim 9, further comprising:
(a) an isolated nucleic acid molecule comprising a nucleotide sequence which encodes a polypeptide having an amino acid sequence at least 95% identical to SEQ ID NO:4; or
(b) an isolated nucleic acid molecule comprising a nucleotide sequence which encodes a polypeptide having an amino acid sequence at least 95% identical to SEQ ID NO:10.

11. The bacterial cell of claim 7;
(a) which is of the genus *Klebsiella;* or
(b) which is deficient in endogenous 2,5-DKG activity.

12. The bacterial cell of claim 11(b), comprising an isolated nucleic acid molecule encoding a polypeptide having at least 80% identity to SEQ ID NO:14 and 2-keto reductase activity.

13. The bacterial cell of claim 11(b), comprising an isolated nucleic acid molecule encoding a polypeptide having at least 80% identity to SEQ ID NO:16 and 5-keto reductase activity.

14. The bacterial cell of claim 7;
(a) which is of the genus *Pantoea;* or
(b) which expresses an enzyme that catalyzes the conversion of 2,5-DKG to 2-KLG.

15. The bacterial cell of claim 14(b), which expresses enzymes that catalyze the conversion of glucose to 2,5-DKG.

16. The bacterial cell of claim 15, which is deficient in endogenase 2-keto-reductase activity.

17. A method of making the isolated nucleic acid molecule of claim 1, comprising introducing into a bacterial cell deficient in endogenous 2,5-DKG permease activity one or more expressible nucleic acid molecules, identifying a cell having 2,5-DKG permease activity following said introduction, and isolating the introduced nucleic acid molecule from said cell.

18. The method of claim 17, wherein said one or more isolated nucleic acid molecules is a genomic DNA library.

19. The method of claim 18, wherein said genomic DNA library is prepared from an environmental sample.

20. The method of claim 17, wherein said bacterial cell is a *Klebsiella oxytoca* deficient in *yiaX2.*

21. The method of claim 17, wherein said bacterial cell comprises an isolated nucleic acid molecule encoding a polypeptide having at least 80% identity to SEQ ID NO:14 and 2-keto reductase activity, and a polypeptide having at least 80% identity to SEQ ID NO:16 and 5-keto reductase activity.

22. A method of using the isolated nucleic acid molecule of claim 1 to enhance 2-KLG production, comprising expressing the polypeptide encoded by said nucleic acid molecule in a bacterial cell which expresses an enzyme that catalyzes the conversion of 2,5-DKG to 2-KLG, wherein the bacterial cell further expresses enzymes that catalyse the conversion of glucose to 2,5-DKG.

23. The method of claim 22, wherein said bacterial cell is deficient in endogenase 2-keto reductase activity.

24. The method of claim 22, wherein said bacterial cell is of the genus Pantoea.

25. The method of claim 22, further comprising converting said 2-KLG to ascorbic acid.

26. An isolated polypeptide which has 2,5-DKG permease activity, said polypeptide comprising:
(a) an amino acid sequence having at least 80% identify to an amino acid sequence selected from the group consisting of SEQ ID NOS:2, 4, 6, 8 and 10; or
(b) an amino acid sequence selected from the group consisting of SEQ ID NOS:2, 4, 6, 8 and 10.

27. An antibody specific for an isolated polypeptide which has 2,5-DKG permease activity, said polypeptide, comprising an amino acid sequence selected from the group consisting of SEQ ID NOs: 2, 4, 6, 8 and 10.

## Patentansprüche

1. Isoliertes Nucleinsäuremolekül, das für ein Polypeptid kodiert, das 2,5-DKG-Permeaseaktivität aufweist, wobei das Nucleinsäuremolekül
(a) eine Nucleotidsequenz mit einer zumindest 80%igen Identität mit einer Nucleotidsequenz, die aus der Gruppe ausgewählt ist, die aus Seq.-ID Nr. 1, 3, 5, 7 und 9 besteht, oder
(b) eine Nucleotidsequenz, die aus der Gruppe ausgewählt ist, die aus Seq.-ID Nr. 1, 3, 5, 7 und 9 besteht, oder
(c) eine Nucleotidsequenz, die für ein Polypeptid mit einer zumindest 80%igen Identität mit einer Aminosäuresequenz kodiert, die aus der Gruppe ausgewählt ist, die aus Seq.-ID Nr. 2, 4, 6, 8 und 10 besteht, umfasst.

2. Isoliertes Nucleinsäuremolekül nach Anspruch 1, das für ein Polypeptid mit einer Aminosäuresequenz kodiert, die aus der Gruppe ausgewählt ist, die aus Seq.-ID Nr. 2, 4, 6, 8 und 10 besteht.

3. Isoliertes Nucleinsäuremolekül nach Anspruch 1, das operativ an einen Promotor der Genexpression gebunden ist.

4. Isoliertes Nucleinsäuremolekül nach Anspruch 3, worin der Promotor ein lac-Promotor ist.

5. Vektor, der das isolierte Nucleinsäuremolekül nach Anspruch 3 oder Anspruch 4 umfasst.

6. Vektor nach Anspruch 5, der ein Spectinomycin-Resistenzgen umfasst.

7. Bakterielle Zelle, die den Vektor nach Anspruch 5 oder Anspruch 6 umfasst.

8. Bakterielle Zelle nach Anspruch 7, worin das isolierte Nucleinsäuremolekül eine Nucleotidsequenz umfasst, die für ein Polypeptid kodiert, das eine 2,5-DKG-Permeaseaktivität aufweist, wobei das Polypeptid eine Aminosäuresequenz mit einer zumindest 80%igen Identität zu einer Aminosäuresequenz aufweist, die aus der Gruppe ausgewählt ist, die aus Seq.-ID Nr. 2, 4, 6, 8 und 10 besteht.

9. Bakterielle Zelle nach Anspruch 8, worin die Aminosäuresequenz zumindest zu 95 % identisch mit Seq.-ID Nr. 8 ist.

10. Bakterielle Zelle nach Anspruch 9, die weiters Folgendes umfasst:
(a) ein isoliertes Nucleinsäuremolekül, das eine Nucleotidsequenz umfasst, die für ein Polypeptid mit einer Aminosäuresequenz kodiert, die zumindest zu 95 % identisch mit Seq.-ID Nr. 4 ist; oder
(b) ein isoliertes Nucleinsäuremolekül, das eine Nucleotidsequenz umfasst, die für ein Polypeptid kodiert, das eine Aminosäuresequenz umfasst, die zumindest zu 95 % identisch mit Seq.-ID Nr. 10 ist.

11. Bakterielle Zelle nach Anspruch 7;
(a) die von der Gattung der Klebsiella abstammt; oder
(b) der die endogene 2,5-DKG-Aktivität fehlt.

12. Bakterielle Zelle nach Anspruch 11 (b), welche ein isoliertes Nucleinsäuremolekül umfasst, das für ein Polypeptid kodiert, das zumindest eine 80%ige Identität mit Seq.-ID Nr. 16 und eine 5-Keto-Reduktase-Aktivität aufweist.

13. Bakterielle Zelle nach Anspruch 11 (b), welche ein isoliertes Nucleinsäuremolekül aufweist, das für ein Polypeptid kodiert, das zumindest 80%ige Identität mit Seq.-ID Nr. 16 und eine 5-Keto-Reduktase-Aktivität aufweist.

14. Bakterielle Zelle nach Anspruch 7;
(a) die von der Gattung der Pantoea abstammt; oder
(b) die ein Enzym exprimiert, das die Umsetzung von 2,5-DKG zu 2-KLG katalysiert.

15. Bakterielle Zelle nach Anspruch 14 (b), welche die Enzyme exprimiert, welche die Umsetzung von Glucose zu 2,5-DKG katalysieren.

16. Bakterielle Zelle nach Anspruch 15, der die Endogenase-2-Keto-Reduktase-Aktivität fehlt.

17. Verfahren zur Herstellung des isolierten Nucleinsäuremoleküls nach Anspruch 1, welches das Einführen von einem oder von mehreren exprimierbaren Nucleinsäuremolekülen in eine bakterielle Zelle, der endogene 2,5-DKG-Permeaseaktivität fehlt, das Identifizieren einer Zelle, die eine 2,5-DKG-Permeaseaktivität nach der Einführung aufweist, und das Isolieren des eingeführten Nucleinsäuremoleküls von der Zelle umfasst.

18. Verfahren nach Anspruch 17, worin das eine oder die mehreren isolierten Nucleinsäuremolekül(e) eine genomische DNA-Bibliothek ist/sind.

19. Verfahren nach Anspruch 18, worin die genomische DNA-Bibliothek aus einer Umweltprobe hergestellt ist.

20. Verfahren nach Anspruch 17, worin die bakterielle Zelle eine Klebsiella oxytoca ist, der yiaX2 fehlt.

21. Verfahren nach Anspruch 17, worin die bakterielle Zelle ein isoliertes Nucleinsäuremolekül, das für ein Polypeptid kodiert, das eine zumindest 80%ige Identität mit Seq.-ID Nr. 14 und 2-Keto-Reductase-Aktivität aufweist, und ein Polypeptid mit einer zumindest 80%igen Identität mit Seq.-ID Nr. 16 und 5-Keto-Reduktase-Aktivität umfasst.

22. Verfahren zur Verwendung des isolierten Nucleinsäuremoleküls nach Anspruch 1, um 2-KLG-Produktion zu verstärken, welches das Exprimieren des Polypeptids, für das das Nucleinsäuremolekül kodiert, in einer bakteriellen Zelle umfasst, die ein Enzym exprimiert, das die Umsetzung von 2,5-DKG zu 2-KLG katalysiert, worin die bakterielle Zelle weiters Enzyme exprimiert, welche die Umsetzung von Glucose zu 2,5-DKG katalysieren.

23. Verfahren nach Anspruch 22, worin der bakteriellen Zelle Endogenase-2-Keto-Reduktaseaktivität fehlt.

24. Verfahren nach Anspruch 22, worin die bakterielle Zelle von der Gattung der Pantoea abstammt.

25. Verfahren nach Anspruch 22, das weiters das 2-KLG zu Ascorbinsäure umsetzt.

26. Isoliertes Polypeptid, das 2,5-DKG-Permeaseaktivität aufweist, wobei das Polypeptid Folgendes umfasst:
(a) eine Aminosäuresequenz mit einer zumindest 80%igen Identität mit einer Aminosäuresequenz, die aus der Gruppe ausgewählt ist, die aus Seq.-ID Nr. 2, 4, 6, 8 und 10 besteht, oder
(b) eine Aminosäuresequenz, die aus der Gruppe ausgewählt ist, die aus Seq.-ID Nr. 2, 4, 6, 8 und 10 besteht.

27. Antikörper, der spezifisch für ein isoliertes Polypeptid ist, das eine 2,5-DKG-Permeaseaktivität aufweist, wobei das Polypeptid eine Aminosäuresequenz umfasst, die aus der Gruppe ausgewählt ist, die aus Seq.-ID Nr. 2, 4, 6, 8 und 10 besteht.

## Revendications

1. Molécule d'acide nucléique isolé codant pour un polypeptide qui a une activité de 2,5-DKG perméase, ladite molécule d'acide nucléique comprenant
(a) une séquence de nucléotides ayant au moins 80% d'identité avec une séquence de nucléotides sélectionnée dans le groupe consistant en SEQ ID NOS:1, 3, 5, 7 et 9; ou
(b) une séquence de nucléotides sélectionnée dans le groupe consistant en SEQ ID NOS:1, 3, 5, 7 et 9; ou
(c) une séquence de nucléotides qui code pour un polypeptide ayant au moins 80% d'identité avec une séquence d'acides aminés sélectionnée dans le groupe consistant en SEQ ID NOS: 2, 4, 6, 8 et 10.

2. Molécule d'acide nucléique isolé selon la revendication 1, qui code pour un polypeptide ayant une séquence d'acides aminés sélectionnée dans le groupe consistant en SEQ ID NO:2, 4, 6, 8 et 10.

3. Molécule d'acide nucléique isolé selon la revendication 1, fonctionnellement liée à un promoteur d'une expression de gènes.

4. Molécule d'acide nucléique isolé selon la revendication 3, où ledit promoteur est un promoteur lac.

5. Vecteur comprenant la molécule d'acide nucléique isolé selon la revendication 3 ou la revendication 4.

6. Vecteur selon la revendication 5, comprenant un gène de résistance à la spectinomycine.

7. Cellule bactérienne comprenant le vecteur de la revendication 5 ou de la revendication 6.

8. Cellule bactérienne selon la revendication 7, où ladite molécule d'acide nucléique isolé comprend une séquence de nucléotides qui code pour un polypeptide qui a une activité de 2,5-DKG perméase, ledit polypeptide ayant une séquence d'acides aminés au moins à 80% identique à une séquence d'acides aminés sélectionnée dans le groupe consistant en SEQ ID NOs:2, 4, 6, 8 et 10.

9. Cellule bactérienne selon la revendication 8, où ladite séquence d'acides aminés est au moins à 95% identique à la SEQ ID NO:8.

10. Cellule bactérienne selon la revendication 9, comprenant en outre:
(a) une molécule d'acide nucléique isolé comprenant une séquence de nucléotides qui code pour un polypeptide ayant une séquence d'acides aminés au moins à 95% identique à la SEQ ID NO:4; ou
(b) une molécule d'acide nucléique isolé comprenant une séquence de nucléotides qui code pour un polypeptide ayant une séquence d'acides aminés au moins à 95% identique à la SEQ ID NO:10.

11. Cellule bactérienne selon la revendication 7,
(a) qui est du gène *Klebsiella;* ou
(b) qui est déficiente dans l'activité de 2,5-DKG endogène.

12. Cellule bactérienne selon la revendication 11 (b), comprenant une molécule d'acide nucléique isolé codant pour un polypeptide ayant au moins 80% d'identité avec la SEQ ID NO:14 et une activité de 2-céto réductase.

13. Cellule bactérienne selon la revendication 11(b), comprenant une molécule d'acide nucléique isolé codant pour un polypeptide ayant au moins 80% d'identité avec la SEQ ID NO: 16 et une activité de 5-céto réductase.

14. Cellule bactérienne selon la revendication 7,
(a) qui est du gène *Pantoea;* ou
(b) qui exprime une enzyme qui catalyse la conversion de 2,5-DKG en 2-KLG.

15. Cellule bactérienne selon la revendication 14(b), qui exprime des enzymes qui catalysent la conversion du glucose en 2,5-DKG.

16. Cellule bactérienne selon la revendication 15, qui est déficiente dans l'activité d'endogène 2-céto-réductase.

17. Méthode de réalisation de la molécule d'acide nucléique isolé selon la revendication 1, comprenant l'introduction dans une cellule bactérienne ayant une déficience en activité perméase endogène 2,5-DKG d'une ou de plusieurs molécules d'acide nucléique apte à être exprimées, identifier une cellule ayant une activité perméase 2,5-DKG à la suite de ladite introduction et isoler la molécule d'acide nucléique introduite de ladite cellule.

18. Méthode selon la revendication 17, dans laquelle un ou plusieurs molécules d'acide nucléique isolé est une biobliothèque d'ADN génomique.

19. Méthode selon la revendication 18, dans laquelle ladite bibliothèque d'ADN génomique est préparée à partir d'un échantillon environnemental.

20. Méthode selon la revendication 17, dans laquelle ladite cellule bactérienne est une *Klebsiella oxytoca* déficiente en *yiaX2.*

21. Méthode selon la revendication 17, où ladite cellule bactérienne comprend une molécule d'acide nucléique isolé codant pour un polypeptide ayant au moins 80% d'identité avec la SEQ ID NO:14 et une activité de 2-céto réductase, et un polypeptide ayant au moins 80% d'identité avec la SEQ ID NO:16 et une activité de 5-céto réductase.

22. Méthode d'utilisation de la molécule d'acide nucléique isolé selon la revendication 1 pour augmenter la production de 2-KLG, comprenant l'expression du polypeptide codé par ladite molécule d'acide nucléique dans une cellule bactérienne qui exprime une enzyme qui catalyse la conversion de 2,5-DKG en 2-KLG, où la cellule bactérienne exprime en outre des enzymes qui catalysent la conversion de glucose en 2,5-DKG.

23. Méthode selon la revendication 22, où ladite cellule bactérienne est déficiente en activité endogène 2-céto-réductase.

24. Méthode selon la revendication 22, où ladite cellule bactérienne est du gène *Pantoea.*

25. Méthode selon la revendication 22, comprenant en outre la conversion de 2-KGL en acide ascorbique.

26. Polypeptide isolé qui a une activité perméase 2,5-DKG, ledit polypeptide comprenant:
(a) une séquence d'acides aminés ayant au moins 80% d'identité avec une séquence d'acides aminés sélectionnée dans le groupe consistant en SEQ ID NOS: 2, 4, 6, 8 et 10; ou
(b) une séquence d'acides aminés sélectionnée dans le groupe consistant en SEQ ID NOS: 2, 4, 6, 8 et 10.

27. Anticorps spécifique pour un polypeptide isolé qui a une activité perméase 2,5-DKG, ledit polypeptide comprenant une séquence d'acides aminés sélectionnée dans le groupe consistant en SEQ ID NOS: 2, 4, 6, 8 et 10.


### SEQUENCE LISTING

<110> Dartois, Veronique A.
   Hoch, James A.
   Valle, Fernando
   Kumar, Manoj
   Microgenomics, Inc.
   Genencor International, Inc.
<120> 2, 5-DKG PERMEASES
<130> FP-SR 4773
<150> US 09/633,294
   <151> 2000-08-04
<150> US 09/677,032
   <151> 2000-09-29
<160> 22
<170> FastSEQ for Windows Version 4.0
<210> 1
   <211> 1500
   <212> DNA
   <213> Unknown
<220>
   <223> environmental source
<221> CDS
   <222> (94)...(1374)
<400> 1
   ggcgaatagc ccggccggcg tcataataac ggccttctct gtaccctaca tacggcggcg 60
   gcgtcatgaa cctcaacttt agtaggcaag cct atg aac agc tct acc aat gca  114
                                        Met Asn Ser Ser Thr Asn Ala
                                         1               5
   acg aaa cgc tgg tgg tac atc atg cct atc gtg ttt atc acg tat agc   162
   Thr Lys Arg Trp Trp Tyr Ile Met Pro Ile Val Phe Ile Thr Tyr Ser
           10                  15                  20
   ctg gcg tat ctc gac cgc gca aac ttc agc ttt gct tcg gca gcg ggc   210
   Leu Ala Tyr Leu Asp Arg Ala Asn Phe Ser Phe Ala Ser Ala Ala Gly
       25                  30                  35
   att acg gaa gat tta ggc att acc aaa ggc atc tcg tcg ctt ctt ggc   258
   Ile Thr Glu Asp Leu Gly Ile Thr Lys Gly Ile Ser Ser Leu Leu Gly
    40                  45                  50                  55
   gca ctt ttc ttc ctc ggc tat ttc ttc ttc cag atc ccg ggg gcg att   306
   Ala Leu Phe Phe Leu Gly Tyr Phe Phe Phe Gln Ile Pro Gly Ala Ile
                   60                  65                  70
   tac gcg gaa cgc cgt agc gta cgg aag ctg att ttc atc tgt ctg atc    354
   Tyr Ala Glu Arg Arg Ser Val Arg Lys Leu Ile Phe Ile Cys Leu Ile
               75                  80                  85
   ctg tgg ggc gcc tgc gcc tcg ctt gac cgg gat ggt gca caa tat tcc    402
   Leu Trp Gly Ala Cys Ala Ser Leu Asp Arg Asp Gly Ala Gln Tyr Ser
           90                  95                  100
   agc gct ggc tgg cga tcc gtt tta ttc tcg gct gtc gtg gaa gcg gcg    450
   Ser Ala Gly Trp Arg Ser Val Leu Phe Ser Ala Val Val Glu Ala Ala
       105                 110                 115
   gtc atg ccg gcg atg ctg att tac atc agt aac tgg ttt acc aaa tca    498
   Val Met Pro Ala Met Leu Ile Tyr Ile Ser Asn Trp Phe Thr Lys Ser
   120                 125                 130                 135
   gaa cgt tca cgc gcc aac acc ttc tta atc ctc ggc aac ccg gtc acg    546
   Glu Arg Ser Arg Ala Asn Thr Phe Leu Ile Leu Gly Asn Pro Val Thr
                   140                 145                 150
   gta ctg tgg atg tcg gtg gtc tcc ggc tac ctg att cag tcc ttc ggc    594
   Val Leu Trp Met Ser Val Val Ser Gly Tyr Leu Ile Gln Ser Phe Gly
               155                 160                 165
   tgg cgt gaa atg ttt att att gaa ggc gtt ccg gcc gtc ctc tgg gcc    642
   Trp Arg Glu Met Phe Ile Ile Glu Gly Val Pro Ala Val Leu Trp Ala
           170                 175                 180
   ttc tgc tgg tgg gtg ctg gtc aaa gtt aaa ccg tcg cag gtg aac tgg    690
   Phe Cys Trp Trp Val Leu Val Lys Val Lys Pro Ser Gln Val Asn Trp
       185                 190                 195
   ttg tcg gaa aac gag aaa gcc gcg ctg cag gcg cag ctg gag agc gag    738
   Leu Ser Glu Asn Glu Lys Ala Ala Leu Gln Ala Gln Leu Glu Ser Glu
   200                 205                 210                 215
   cag cag ggt att aaa gcc gtg cgt aac tac ggc gaa gcc ttc cgc tca    786
   Gln Gln Gly Ile Lys Ala Val Arg Asn Tyr Gly Glu Ala Phe Arg Ser
                   220                 225                 230
   cgc aac gtc att cta ctg tgc atg cag tat ttt gcc tgg agt atc ggc    834
   Arg Asn Val Ile Leu Leu Cys Met Gln Tyr Phe Ala Trp Ser Ile Gly
               235                 240                 245
   gtg tac ggt ttt gtg ctg tgg ttg ccg tca att att cgc agc ggc ggc    882
   Val Tyr Gly Phe Val Leu Trp Leu Pro Ser Ile Ile Arg Ser Gly Gly
           250                 255                 260
   gtc aat atg ggg atg gtg gaa gtc ggc tgg ctc tct tcg gtg cct tat    930
   Val Asn Met Gly Met Val Glu Val Gly Trp Leu Ser Ser Val Pro Tyr
       265                 270                 275
   ctg gcc gcg act att gcg atg atc gtc gtc tcc tgg gct tcc gat aaa    978
   Leu Ala Ala Thr Ile Ala Met Ile Val Val Ser Trp Ala Ser Asp Lys
   280                 285                 290                 295
   atg cag aac cgt aaa ctg ttc gtc tgg ccg ctg ctg ctg att ggc gga   1026
   Met Gln Asn Arg Lys Leu Phe Val Trp Pro Leu Leu Leu Ile Gly Gly
                   300                 305                 310
   ctg gct ttt att ggc tca tgg gcc gtc ggc gct aac cat ttc tgg gcc   1074
   Leu Ala Phe Ile Gly Ser Trp Ala Val Gly Ala Asn His Phe Trp Ala
               315                 320                 325
   tct tat acc ctg ctg gtg att gcc aat gcg gca atg tac gcc cct tac   1122
   Ser Tyr Thr Leu Leu Val Ile Ala Asn Ala Ala Met Tyr Ala Pro Tyr
           330                 335                 340
   ggt ccg ttt ttc gcc atc att ccg gaa atg ctg ccg cgt aac gtc gcc   1170
   Gly Pro Phe Phe Ala Ile Ile Pro Glu Met Leu Pro Arg Asn Val Ala
       345                 350                 355
   ggt ggc gca atg gcg ctc atc aac agc atg ggg gcc tta ggt tca ttc   1218
   Gly Gly Ala Met Ala Leu Ile Asn Ser Met Gly Ala Leu Gly Ser Phe
   360                 365                 370                 375
   ttt ggt tcg tgg ttc gtg ggc tac ctg aac ggc acc acc ggc agt cca   1266
   Phe Gly Ser Trp Phe Val Gly Tyr Leu Asn Gly Thr Thr Gly Ser Pro
                   380                 385                 390
   tca gcc tca tac att ttc atg gga gtg gcg ctt ttc gcc tcg gta tgg   1314
   Ser Ala Ser Tyr Ile Phe Met Gly Val Ala Leu Phe Ala Ser Val Trp
               395                 400                 405
   ctt act tta att gtt aag cct gct aac aat caa aag ctc ccc atc ggc   1362
   Leu Thr Leu Ile Val Lys Pro Ala Asn Asn Gln Lys Leu Pro Ile Gly
           410                 415                 420
   gct cgt cac gcc tgacctttac tacttacgga gatcacgcct tgggtacgtt       1414
   Ala Arg His Ala
       425
   gcaggacaaa ccgataggca ccgcaaaggc tggggccatc gagcagcgcg taaacagtca 1474
   gctggttgct gtcgctgtgc ggcgtc                                      1500
<210> 2
   <211> 427
   <212> PRT
   <213> Unknown
<220>
   <223> environmental source
<400> 2
   Met Asn Ser Ser Thr Asn Ala Thr Lys Arg Trp Trp Tyr Ile Met Pro
    1               5                  10                  15
   Ile Val Phe Ile Thr Tyr Ser Leu Ala Tyr Leu Asp Arg Ala Asn Phe
               20                  25                  30
   Ser Phe Ala Ser Ala Ala Gly Ile Thr Glu Asp Leu Gly Ile Thr Lys
           35                  40                  45
   Gly Ile Ser Ser Leu Leu Gly Ala Leu Phe Phe Leu Gly Tyr Phe Phe
       50                  55                  60
   Phe Gln Ile Pro Gly Ala Ile Tyr Ala Glu Arg Arg Ser Val Arg Lys
   65                  70                  75                  80
   Leu Ile Phe Ile Cys Leu Ile Leu Trp Gly Ala Cys Ala Ser Leu Asp
                   85                  90                  95
   Arg Asp Gly Ala Gln Tyr Ser Ser Ala Gly Trp Arg Ser Val Leu Phe
               100                 105                 110
   Ser Ala Val Val Glu Ala Ala Val Met Pro Ala Met Leu Ile Tyr Ile
           115                 120                 125
   Ser Asn Trp Phe Thr Lys Ser Glu Arg Ser Arg Ala Asn Thr Phe Leu
       130                 135                 140
   Ile Leu Gly Asn Pro Val Thr Val Leu Trp Met Ser Val Val Ser Gly
   145                 150                 155                 160
   Tyr Leu Ile Gln Ser Phe Gly Trp Arg Glu Met Phe Ile Ile Glu Gly
                   165                 170                 175
   Val Pro Ala Val Leu Trp Ala Phe Cys Trp Trp Val Leu Val Lys Val
               180                 185                 190
   Lys Pro Ser Gln Val Asn Trp Leu Ser Glu Asn Glu Lys Ala Ala Leu
           195                 200                 205
   Gln Ala Gln Leu Glu Ser Glu Gln Gln Gly Ile Lys Ala Val Arg Asn
       210                 215                 220
   Tyr Gly Glu Ala Phe Arg Ser Arg Asn Val Ile Leu Leu Cys Met Gln
   225                 230                 235                 240
   Tyr Phe Ala Trp Ser Ile Gly Val Tyr Gly Phe Val Leu Trp Leu Pro
                   245                 250                 255
   Ser Ile Ile Arg Ser Gly Gly Val Asn Met Gly Met Val Glu Val Gly
               260                 265                 270
   Trp Leu Ser Ser Val Pro Tyr Leu Ala Ala Thr Ile Ala Met Ile Val
           275                 280                 285
   Val Ser Trp Ala Ser Asp Lys Met Gln Asn Arg Lys Leu Phe Val Trp
       290                 295                 300
   Pro Leu Leu Leu Ile Gly Gly Leu Ala Phe Ile Gly Ser Trp Ala Val
   305                 310                 315                 320
   Gly Ala Asn His Phe Trp Ala Ser Tyr Thr Leu Leu Val Ile Ala Asn
                   325                 330                 335
   Ala Ala Met Tyr Ala Pro Tyr Gly Pro Phe Phe Ala Ile Ile Pro Glu
               340                 345                 350
   Met Leu Pro Arg Asn Val Ala Gly Gly Ala Met Ala Leu Ile Asn Ser
           355                 360                 365
   Met Gly Ala Leu Gly Ser Phe Phe Gly Ser Trp Phe Val Gly Tyr Leu
       370                 375                 380
   Asn Gly Thr Thr Gly Ser Pro Ser Ala Ser Tyr Ile Phe Met Gly Val
   385                 390                 395                 400
   Ala Leu Phe Ala Ser Val Trp Leu Thr Leu Ile Val Lys Pro Ala Asn
                   405                 410                 415
   Asn Gln Lys Leu Pro Ile Gly Ala Arg His Ala
               420                 425
<210> 3
   <211> 1775
   <212> DNA
   <213> Unknown
<220>
   <223> environmental source
<221> CDS
   <222> (214)...(1491)
<400> 3
   ggcaatttgc ggtgtttttt ccgcaggacg ttcatcgtcc ggcctgtatt catcaacggc      60
   cctgcgctat tcgcaaagtg gtggtgaaaa taccgctgcg ttatttaacg cccaataagc     120
   aacaccgagt ttataaccct gaacgacacg gctgcgggcc tgtgtagacg cccctacgcc     180
   ttaacaccac taaatgactc tacaggtgta tat atg aat aca gcc tct gtt tct      234
                                        Met Asn Thr Ala Ser Val Ser
                                         1               5
   gtc acc caa agc cag gcg atc ccc aaa tta cgc tgg ttg aga ata gtg       282
   Val Thr Gln Ser Gln Ala Ile Pro Lys Leu Arg Trp Leu Arg Ile Val
           10                  15                  20
   ccg cct att ctt att acc tgc att att tcc tat atg gac cgg gtg aac       330
   Pro Pro Ile Leu Ile Thr Cys Ile Ile Ser Tyr Met Asp Arg Val Asn
       25                  30                  35
   atc gcc ttc gcc atg ccc ggc ggc atg gac gat gaa ctg ggc atc acc       378
   Ile Ala Phe Ala Met Pro Gly Gly Met Asp Asp Glu Leu Gly Ile Thr
    40                  45                  50                  55
   gcc tcg atg gcc ggg ttg gcc ggc ggt att ttc ttt atc ggt tat ctg       426
   Ala Ser Met Ala Gly Leu Ala Gly Gly Ile Phe Phe Ile Gly Tyr Leu
                   60                  65                  70
   ttc ttg cag gta ccc ggc ggc aag ctg gcg gtg tac ggc aac ggc aag       474
   Phe Leu Gln Val Pro Gly Gly Lys Leu Ala Val Tyr Gly Asn Gly Lys
               75                  80                  85
   aaa ttc atc ggt tgg tcg ttg ttg gcc tgg gcg gtg att tcc gtg ctg       522
   Lys Phe Ile Gly Trp Ser Leu Leu Ala Trp Ala Val Ile Ser Val Leu
           90                  95                  100
   acc ggg ctg gtc acg aat cag tat caa ttg ctg ttc ctg cgc ttc gcc       570
   Thr Gly Leu Val Thr Asn Gln Tyr Gln Leu Leu Phe Leu Arg Phe Ala
       105                 110                 115
   ctc ggc cgt ttc cga agc ggc atg ctg cgg tgg gtg ctg acc atg atc       618
   Leu Gly Arg Phe Arg Ser Gly Met Leu Arg Trp Val Leu Thr Met Ile
   120                 125                 130                 135
   agc aac tgg ttc ccg gac aag gaa cgc ggg cgc gcc aac gcc atc gtc       666
   Ser Asn Trp Phe Pro Asp Lys Glu Arg Gly Arg Ala Asn Ala Ile Val
                   140                 145                 150
   atc atg ttc gtg ccg atc gcc ggc atc ctt acc gca ccg ctg tcc ggc       714
   Ile Met Phe Val Pro Ile Ala Gly Ile Leu Thr Ala Pro Leu Ser Gly
               155                 160                 165
   tgg atc atc acc gcc tgg gac tgg cgc atg ctg ttc ctg gtc gag ggc      762
   Trp Ile Ile Thr Ala Trp Asp Trp Arg Met Leu Phe Leu Val Glu Gly
           170                 175                 180
   gcg ctg tcg ctg gtc gtg atg gtg ctg tgg tat ttc acc atc agc aac      810
   Ala Leu Ser Leu Val Val Met Val Leu Trp Tyr Phe Thr Ile Ser Asn
       185                 190                 195
   cgt cca caa gag gcc aaa agg att tcg cag gcg gaa aaa gat tat ctg      858
   Arg Pro Gln Glu Ala Lys Arg Ile Ser Gln Ala Glu Lys Asp Tyr Leu
   200                 205                 210                 215
   atc aaa acg ctg cac gac gaa cag ttg ctg atc aaa ggc aaa acg gtg      906
   Ile Lys Thr Leu His Asp Glu Gln Leu Leu Ile Lys Gly Lys Thr Val
                   220                 225                 230
   cgc aac gcc tcg ctg cgt cgg gtg ctg ggc gac aaa atc atg tgg aag      954
   Arg Asn Ala Ser Leu Arg Arg Val Leu Gly Asp Lys Ile Met Trp Lys
               235                 240                 245
   ttc ttc tac cag acc ggg ata tac ggc tac acc ctg tgg ctg ccg acc     1002
   Phe Phe Tyr Gln Thr Gly Ile Tyr Gly Tyr Thr Leu Trp Leu Pro Thr
           250                 255                 260
   att ctc aag ggg ctc acc aac ggc aat atg gag cag gtc ggg atg ctg     1050
   Ile Leu Lys Gly Leu Thr Asn Gly Asn Met Glu Gln Val Gly Met Leu
       265                 270                 275
   gct atc ctg ccc tat atc ggc gcc atc ttc ggc atg ctg atc att tcc     1098
   Ala Ile Leu Pro Tyr Ile Gly Ala Ile Phe Gly Met Leu Ile Ile Ser
   280                 285                 290                 295
   acc ctc tcc gat cgc acc ggc aag cgc aaa gtg ttc gtc gca ctg ccg     1146
   Thr Leu Ser Asp Arg Thr Gly Lys Arg Lys Val Phe Val Ala Leu Pro
                   300                 305                 310
   ctg gcc tgc ttt gcc atc tgc atg gcg ctg tcg gtg ctg ctg aag gat     1194
   Leu Ala Cys Phe Ala Ile Cys Met Ala Leu Ser Val Leu Leu Lys Asp
               315                 320                 325
   cac atc tgg tgg tcg tac gcg gcg ctg gtg ggc tgt ggc gtc ttt acc     1242
   His Ile Trp Trp Ser Tyr Ala Ala Leu Val Gly Cys Gly Val Phe Thr
           330                 335                 340
   cag gcc gcc gcc ggg gtg ttc tgg acc att ccg ccc aag ctg ttt aac     1290
   Gln Ala Ala Ala Gly Val Phe Trp Thr Ile Pro Pro Lys Leu Phe Asn
       345                 350                 355
   gcc gaa atg gcc ggc ggc gcg cgc ggc gtg atc aat gca ctg ggc aac     1338
   Ala Glu Met Ala Gly Gly Ala Arg Gly Val Ile Asn Ala Leu Gly Asn
   360                 365                 370                 375
   ctc ggc ggt ttc tgc ggc ccc tat atg gtc ggc gtg ttg atc acc ttg     1386
   Leu Gly Gly Phe Cys Gly Pro Tyr Met Val Gly Val Leu Ile Thr Leu
                   380                 385                 390
   ttc agc aaa gac gtc ggc gtt tac agc ctc gcg gtg tcg ctg gcc tcc     1434
   Phe Ser Lys Asp Val Gly Val Tyr Ser Leu Ala Val Ser Leu Ala Ser
               395                 400                 405
   gcc tcg gtg ctg gcg ttg atg ctg ccg aac aga tgc gac caa aaa gcg     1482
   Ala Ser Val Leu Ala Leu Met Leu Pro Asn Arg Cys Asp Gln Lys Ala
           410                 415                 420
   ggg gcc gaa taatggacta ttggctgggg ctggactgcg gcggcacctt             1531
   Gly Ala Glu
       425
   tatcaaagcc ggcctgtatg accggaatgg cgcagaactg ggcatagccc gccgtacgct   1591
   ggacattgtc gcgccgcaac ccggctgggc ggaacgtgac atgcccgcgc tgtggcagac   1651
   cgccgccgag gtgatccgcg aattgctggc ccgcaacgac attgccgacg ctgatattca   1711
   ggccatcggc atctcggcgc agggtaaagg cgcgtttttg ttagacgagc aaggccaacc   1771
   gttg                                                                1775
<210> 4
   <211> 426
   <212> PRT
   <213> Unknown
<220>
   <223> environmental source
<400> 4
   Met Asn Thr Ala Ser Val Ser Val Thr Gln Ser Gln Ala Ile Pro Lys
    1               5                  10                  15
   Leu Arg Trp Leu Arg Ile Val Pro Pro Ile Leu Ile Thr Cys Ile Ile
               20                  25                  30
   Ser Tyr Met Asp Arg Val Asn Ile Ala Phe Ala Met Pro Gly Gly Met
           35                  40                  45
   Asp Asp Glu Leu Gly Ile Thr Ala Ser Met Ala Gly Leu Ala Gly Gly
       50                  55                  60
   Ile Phe Phe Ile Gly Tyr Leu Phe Leu Gln Val Pro Gly Gly Lys Leu
   65                  70                  75                  80
   Ala Val Tyr Gly Asn Gly Lys Lys Phe Ile Gly Trp Ser Leu Leu Ala
                   85                  90                  95
   Trp Ala Val Ile Ser Val Leu Thr Gly Leu Val Thr Asn Gln Tyr Gln
               100                 105                 110
   Leu Leu Phe Leu Arg Phe Ala Leu Gly Arg Phe Arg Ser Gly Met Leu
           115                 120                 125
   Arg Trp Val Leu Thr Met Ile Ser Asn Trp Phe Pro Asp Lys Glu Arg
       130                 135                 140
   Gly Arg Ala Asn Ala Ile Val Ile Met Phe Val Pro Ile Ala Gly Ile
   145                 150                 155                 160
   Leu Thr Ala Pro Leu Ser Gly Trp Ile Ile Thr Ala Trp Asp Trp Arg
                   165                 170                 175
   Met Leu Phe Leu Val Glu Gly Ala Leu Ser Leu Val Val Met Val Leu
               180                 185                 190
   Trp Tyr Phe Thr Ile Ser Asn Arg Pro Gln Glu Ala Lys Arg Ile Ser
           195                 200                 205
   Gln Ala Glu Lys Asp Tyr Leu Ile Lys Thr Leu His Asp Glu Gln Leu
       210                 215                 220
   Leu Ile Lys Gly Lys Thr Val Arg Asn Ala Ser Leu Arg Arg Val Leu
   225                 230                 235                 240
   Gly Asp Lys Ile Met Trp Lys Phe Phe Tyr Gln Thr Gly Ile Tyr Gly
                   245                 250                 255
   Tyr Thr Leu Trp Leu Pro Thr Ile Leu Lys Gly Leu Thr Asn Gly Asn
               260                 265                 270
   Met Glu Gln Val Gly Met Leu Ala Ile Leu Pro Tyr Ile Gly Ala Ile
           275                 280                 285
   Phe Gly Met Leu Ile Ile Ser Thr Leu Ser Asp Arg Thr Gly Lys Arg
       290                 295                 300
   Lys Val Phe Val Ala Leu Pro Leu Ala Cys Phe Ala Ile Cys Met Ala
   305                 310                 315                 320
   Leu Ser Val Leu Leu Lys Asp His Ile Trp Trp Ser Tyr Ala Ala Leu
                   325                 330                 335
   Val Gly Cys Gly Val Phe Thr Gln Ala Ala Ala Gly Val Phe Trp Thr
               340                 345                 350
   Ile Pro Pro Lys Leu Phe Asn Ala Glu Met Ala Gly Gly Ala Arg Gly
           355                 360                 365
   Val Ile Asn Ala Leu Gly Asn Leu Gly Gly Phe Cys Gly Pro Tyr Met
       370                 375                 380
   Val Gly Val Leu Ile Thr Leu Phe Ser Lys Asp Val Gly Val Tyr Ser
   385                 390                 395                 400
   Leu Ala Val Ser Leu Ala Ser Ala Ser Val Leu Ala Leu Met Leu Pro
                   405                 410                 415
   Asn Arg Cys Asp Gln Lys Ala Gly Ala Glu
               420                 425
<210> 5
   <211> 1478
   <212> DNA
   <213> Klebsiella oxytoca
<220>
   <221> CDS
   <222> (73)...(1353)
<400> 5
   ttgcccgccg ccgctgctca gaccatcgat cttttctatg atgagcgtca cctcactcac      60
   agaggcaaac ct atg aat agt tca acg aat gca aca aaa cgc tgg tgg tac     111
                 Met Asn Ser Ser Thr Asn Ala Thr Lys Arg Trp Trp Tyr
                  1               5                  10
   atc atg cct atc gtg ttt atc acg tat agc ctg gcg tac ctc gac cgc       159
   Ile Met Pro Ile Val Phe Ile Thr Tyr Ser Leu Ala Tyr Leu Asp Arg
        15                  20                  25
   gct aac ttc agc ttc gct tcg gcg gcc gga att act gaa gac ctg ggg       207
   Ala Asn Phe Ser Phe Ala Ser Ala Ala Gly Ile Thr Glu Asp Leu Gly
    30                  35                  40                  45
   atc acc aaa ggt atc tcc tcc ctt ctg ggg gcg ctg ttc ttc ctc ggc       255
   Ile Thr Lys Gly Ile Ser Ser Leu Leu Gly Ala Leu Phe Phe Leu Gly
                   50                  55                  60
   tac ttc ttc ttt cag atc ccc ggc gcg att tat gcc gaa cgc cgc agc      303
   Tyr Phe Phe Phe Gln Ile Pro Gly Ala Ile Tyr Ala Glu Arg Arg Ser
               65                  70                  75
   gta cgt aaa ctc att ttc atc tgc ctg atc ctg tgg ggt gcc tgc gcc      351
   Val Arg Lys Leu Ile Phe Ile Cys Leu Ile Leu Trp Gly Ala Cys Ala
           80                  85                  90
   tca ctc gac cgg gat ggt gca caa tat tcc cgc gct ggg cgg gcg atc      399
   Ser Leu Asp Arg Asp Gly Ala Gln Tyr Ser Arg Ala Gly Arg Ala Ile
       95                  100                 105
   cgc ttt atc ctt ggc gtg gtc gag gcc gca gtc atg ccg gcg atg ctg      447
   Arg Phe Ile Leu Gly Val Val Glu Ala Ala Val Met Pro Ala Met Leu
   110                 115                 120                 125
   ata tac atc agc aac tgg ttt acc aaa tcc gaa cgc tcg cgc gcc aat      495
   Ile Tyr Ile Ser Asn Trp Phe Thr Lys Ser Glu Arg Ser Arg Ala Asn
                   130                 135                 140
   acc ttc ctg atc ctc ggc aac ccg gtg acg gtg ctg tgg atg tcg gtg      543
   Thr Phe Leu Ile Leu Gly Asn Pro Val Thr Val Leu Trp Met Ser Val
               145                 150                 155
   gtc tcc ggc tac ctg att cag gct ttc ggc tgg cgg gag atg ttt att      591
   Val Ser Gly Tyr Leu Ile Gln Ala Phe Gly Trp Arg Glu Met Phe Ile
           160                 165                 170
   att gaa ggc gtt ccg gcg gtg att tgg gcc ttc tgc tgg tgg gtg ctg      639
   Ile Glu Gly Val Pro Ala Val Ile Trp Ala Phe Cys Trp Trp Val Leu
       175                 180                 185
   gta aaa gat aaa ccg tct cag gtc aac tgg ctg gcg gaa agc gaa aag      687
   Val Lys Asp Lys Pro Ser Gln Val Asn Trp Leu Ala Glu Ser Glu Lys
   190                 195                 200                 205
   gcc gca ttg cag gag cag ctg gag cgc gaa cag cag ggt atc aaa ccg      735
   Ala Ala Leu Gln Glu Gln Leu Glu Arg Glu Gln Gln Gly Ile Lys Pro
                   210                 215                 220
   gtg cgc aac tac ggt gag gcc ttc cgc tcg cgt aac gtg gtc ctg ctg      783
   Val Arg Asn Tyr Gly Glu Ala Phe Arg Ser Arg Asn Val Val Leu Leu
               225                 230                 235
   tgc atg caa tat ttc gcc tgg agc atc ggg gtt tac ggt ttc gtg ctg      831
   Cys Met Gln Tyr Phe Ala Trp Ser Ile Gly Val Tyr Gly Phe Val Leu
           240                 245                 250
   tgg ctg ccg tca att atc cgc agc ggc ggc gag aat atg ggc atg gtc      879
   Trp Leu Pro Ser Ile Ile Arg Ser Gly Gly Glu Asn Met Gly Met Val
       255                 260                 265
   gag gtc ggc tgg ctc tca tcc gtc ccc tac ctg gcg gca acc atc gcc       927
   Glu Val Gly Trp Leu Ser Ser Val Pro Tyr Leu Ala Ala Thr Ile Ala
   270                 275                 280                 285
   atg atc gtg gtc tcc tgg gcc tcc gat aaa atg cag aac cgc aag cta       975
   Met Ile Val Val Ser Trp Ala Ser Asp Lys Met Gln Asn Arg Lys Leu
                   290                 295                 300
   ttc gtc tgg ccg ctg ctg ctg att gcc gcc ttc gcg ttt att ggc tcc      1023
   Phe Val Trp Pro Leu Leu Leu Ile Ala Ala Phe Ala Phe Ile Gly Ser
               305                 310                 315
   tgg gcc gtc ggc gct aac cat ttc tgg gtc tct tat acc ctg ctg gtc      1071
   Trp Ala Val Gly Ala Asn His Phe Trp Val Ser Tyr Thr Leu Leu Val
           320                 325                 330
   att gcc ggc gcg gcg atg tac gcc ccc tac ggg ccg ttc ttc gcc atc      1119
   Ile Ala Gly Ala Ala Met Tyr Ala Pro Tyr Gly Pro Phe Phe Ala Ile
       335                 340                 345
   att ccc gag atg ctg ccg cgt aac gtc gcc ggg ggc gcc atg gcg ctg      1167
   Ile Pro Glu Met Leu Pro Arg Asn Val Ala Gly Gly Ala Met Ala Leu
   350                 355                 360                 365
   att aac agc atg ggc gcg ctg ggt tca ttc ttt ggc tca tgg ttt gtc      1215
   Ile Asn Ser Met Gly Ala Leu Gly Ser Phe Phe Gly Ser Trp Phe Val
                   370                 375                 380
   ggc tac ctg aac ggc acc acc ggc agc ccg tca gcc tcg tac att ttt      1263
   Gly Tyr Leu Asn Gly Thr Thr Gly Ser Pro Ser Ala Ser Tyr Ile Phe
               385                 390                 395
   atg gga gtg gcg ctt ttc gtc tcg gta tgg ctt act ttg att gtt aag      1311
   Met Gly Val Ala Leu Phe Val Ser Val Trp Leu Thr Leu Ile Val Lys
           400                 405                 410
   cct gct aat aat caa aaa ctt ccg ctc ggc gca cgt cac gcc              1353
   Pro Ala Asn Asn Gln Lys Leu Pro Leu Gly Ala Arg His Ala
       415                 420                 425
   tgaaacatta acgcaacgga gaaccgcatg aagccgtcag tcattctcta caaaacgctt    1413
   cccgacgacc tgcaacaagc gtctggaaca acactttacc gtcacgcagg tgaaaaacct    1473
   gcgtt                                                                1478
<210> 6
   <211> 427
   <212> PRT
   <213> Klebsiella oxytoca
<400> 6
   Met Asn Ser Ser Thr Asn Ala Thr Lys Arg Trp Trp Tyr Ile Met Pro
    1               5                  10                  15
   Ile Val Phe Ile Thr Tyr Ser Leu Ala Tyr Leu Asp Arg Ala Asn Phe
               20                  25                  30
   Ser Phe Ala Ser Ala Ala Gly Ile Thr Glu Asp Leu Gly Ile Thr Lys
           35                  40                  45
   Gly Ile Ser Ser Leu Leu Gly Ala Leu Phe Phe Leu Gly Tyr Phe Phe
       50                  55                  60
   Phe Gln Ile Pro Gly Ala Ile Tyr Ala Glu Arg Arg Ser Val Arg Lys
   65                  70                  75                  80
   Leu Ile Phe Ile Cys Leu Ile Leu Trp Gly Ala Cys Ala Ser Leu Asp
                   85                  90                  95
   Arg Asp Gly Ala Gln Tyr Ser Arg Ala Gly Arg Ala Ile Arg Phe Ile
               100                 105                 110
   Leu Gly Val Val Glu Ala Ala Val Met Pro Ala Met Leu Ile Tyr Ile
           115                 120                 125
   Ser Asn Trp Phe Thr Lys Ser Glu Arg Ser Arg Ala Asn Thr Phe Leu
       130                 135                 140
   Ile Leu Gly Asn Pro Val Thr Val Leu Trp Met Ser Val Val Ser Gly
   145                 150                 155                 160
   Tyr Leu Ile Gln Ala Phe Gly Trp Arg Glu Met Phe Ile Ile Glu Gly
                   165                 170                 175
   Val Pro Ala Val Ile Trp Ala Phe Cys Trp Trp Val Leu Val Lys Asp
               180                 185                 190
   Lys Pro Ser Gln Val Asn Trp Leu Ala Glu Ser Glu Lys Ala Ala Leu
           195                 200                 205
   Gln Glu Gln Leu Glu Arg Glu Gln Gln Gly Ile Lys Pro Val Arg Asn
       210                 215                 220
   Tyr Gly Glu Ala Phe Arg Ser Arg Asn Val Val Leu Leu Cys Met Gln
   225                 230                 235                 240
   Tyr Phe Ala Trp Ser Ile Gly Val Tyr Gly Phe Val Leu Trp Leu Pro
                   245                 250                 255
   Ser Ile Ile Arg Ser Gly Gly Glu Asn Met Gly Met Val Glu Val Gly
               260                 265                 270
   Trp Leu Ser Ser Val Pro Tyr Leu Ala Ala Thr Ile Ala Met Ile Val
           275                 280                 285
   Val Ser Trp Ala Ser Asp Lys Met Gln Asn Arg Lys Leu Phe Val Trp
       290                 295                 300
   Pro Leu Leu Leu Ile Ala Ala Phe Ala Phe Ile Gly Ser Trp Ala Val
   305                 310                 315                 320
   Gly Ala Asn His Phe Trp Val Ser Tyr Thr Leu Leu Val Ile Ala Gly
                   325                 330                 335
   Ala Ala Met Tyr Ala Pro Tyr Gly Pro Phe Phe Ala Ile Ile Pro Glu
               340                 345                 350
   Met Leu Pro Arg Asn Val Ala Gly Gly Ala Met Ala Leu Ile Asn Ser
           355                 360                 365
   Met Gly Ala Leu Gly Ser Phe Phe Gly Ser Trp Phe Val Gly Tyr Leu
       370                 375                 380
   Asn Gly Thr Thr Gly Ser Pro Ser Ala Ser Tyr Ile Phe Met Gly Val
   385                 390                 395                 400
   Ala Leu Phe Val Ser Val Trp Leu Thr Leu Ile Val Lys Pro Ala Asn
                   405                 410                 415
   Asn Gln Lys Leu Pro Leu Gly Ala Arg His Ala
               420                 425
<210> 7
   <211> 1600
   <212> DNA
   <213> Pantoea citrea
<220>
   <221> CDS
   <222> (214)...(1521)
<400> 7
   tctgagcttt tcccgacctt atccctgtca gatccctgcc tggttcacga accttgtaac      60
   acactttctt aacatgccct tacgtgaccc tgatcccaca ctgtcagtgc aaaaacacgt     120
   tttatagctc ctgataagca cagttcgcag cgcgtaactg cacccgcagg gttctgcttt     180
   gcgtcaactg acaaaacaga aaggggatat atc atg caa aaa tca cag ccg gga      234
                                        Met Gln Lys Ser Gln Pro Gly
                                         1               5
   acc cgc tgg ttt cgg att att gtg ccg atc ctg ata gcc tgc atc atg      282
   Thr Arg Trp Phe Arg Ile Ile Val Pro Ile Leu Ile Ala Cys Ile Met
           10                  15                  20
   tcg ttt atg gat cgg gta aat atc agt ttc gca ttg ccg ggc ggt atg      330
   Ser Phe Met Asp Arg Val Asn Ile Ser Phe Ala Leu Pro Gly Gly Met
       25                  30                  35
   gag cag gat ctg ctg atg tcc agc cag atg gcc ggg gta gtt agc ggt      378
   Glu Gln Asp Leu Leu Met Ser Ser Gln Met Ala Gly Val Val Ser Gly
   40                  45                  50                  55
   att ttc ttt att ggt tat ctg ttt ttg cag gtt cct ggt ggg cat atc      426
   Ile Phe Phe Ile Gly Tyr Leu Phe Leu Gln Val Pro Gly Gly His Ile
                   60                  65                  70
   gca gta cgt ggc agt ggt aaa cgt ttt att gcc tgg tcg ctt gtt gcc      474
   Ala Val Arg Gly Ser Gly Lys Arg Phe Ile Ala Trp Ser Leu Val Ala
               75                  80                  85
   tgg gcc gtt gtt tct gtc gct acc ggg ttt gtg act cat cag tac cag      522
   Trp Ala Val Val Ser Val Ala Thr Gly Phe Val Thr His Gln Tyr Gln
           90                  95                  100
   ctg ttg att tta cgt ttt gca ctg ggg gtc tct gaa ggt ggg atg ttg      570
   Leu Leu Ile Leu Arg Phe Ala Leu Gly Val Ser Glu Gly Gly Met Leu
       105                 110                 115
   ccg gta gtt ctg aca atg gtc agc aac tgg ttt cct gaa aaa gag ctg      618
   Pro Val Val Leu Thr Met Val Ser Asn Trp Phe Pro Glu Lys Glu Leu
   120                 125                 130                 135
   ggg cgt gct aat gca ttt gtc atg atg ttc gcc ccg ctt ggc gga atg      666
   Gly Arg Ala Asn Ala Phe Val Met Met Phe Ala Pro Leu Gly Gly Met
                   140                 145                 150
   att acc gcc cct gtc tcc gga tgg att att gca ctg cta gac tgg cgc      714
   Ile Thr Ala Pro Val Ser Gly Trp Ile Ile Ala Leu Leu Asp Trp Arg
               155                 160                 165
   tgg tta ttt att atc gaa gga tta ctg tcg gta gtg gtt ctg gca gtc      762
   Trp Leu Phe Ile Ile Glu Gly Leu Leu Ser Val Val Val Leu Ala Val
   170 175 180
   tgg tgg ctg atg gtc agt gac cgc cct gaa gat gcc cgt tgg ctg ccg 810
   Trp Trp Leu Met Val Ser Asp Arg Pro Glu Asp Ala Arg Trp Leu Pro
   185 190 195
   gca gca gaa cgg gaa tat ctg ctg cgc gaa atg gcc cgt gac aag gcc 858
   Ala Ala Glu Arg Glu Tyr Leu Leu Arg Glu Met Ala Arg Asp Lys Ala
   200 205 210 215
   gag cgg agc aaa ctc cct ccg atc agt cat gct ccc ctg caa gag gtt 906
   Glu Arg Ser Lys Leu Pro Pro Ile Ser His Ala Pro Leu Gln Glu Val
   220 225 230
   ttc cat aac ccg ggc ctg atg aag tta gtg att ctg aac ttt ttc tat 954
   Phe His Asn Pro Gly Leu Met Lys Leu Val Ile Leu Asn Phe Phe Tyr
   235 240 245
   cag aca ggt gat tac gga tac act ctg tgg ctg ccg act att atc aaa 1002
   Gln Thr Gly Asp Tyr Gly Tyr Thr Leu Trp Leu Pro Thr Ile Ile Lys
   250 255 260
   aac ctg acc gga gct agt att ggt aac gtc ggt ttg ctg aca gtg cta 1050
   Asn Leu Thr Gly Ala Ser Ile Gly Asn Val Gly Leu Leu Thr Val Leu
   265 270 275
   cct ttt atc gcg acg tta tca ggg att tat gtc gtc tct tac ctg agc 1098
   Pro Phe Ile Ala Thr Leu Ser Gly Ile Tyr Val Val Ser Tyr Leu Ser
   280 285 290 295
   gat aaa acc ggc aaa cgt cgg caa tgg gtg atg att tct ctg ttc tgt 1146
   Asp Lys Thr Gly Lys Arg Arg Gln Trp Val Met Ile Ser Leu Phe Cys
   300 305 310
   ttt gcg gcc tgc ctg ttg gcc tca gtc ctg tta cgt gaa ttt gtg ctg 1194
   Phe Ala Ala Cys Leu Leu Ala Ser Val Leu Leu Arg Glu Phe Val Leu
   315 320 325
   gct gct tat ctg gct ctg gtg gct tgc ggc ttt ttc ctg aaa gca gcc 1242
   Ala Ala Tyr Leu Ala Leu Val Ala Cys Gly Phe Phe Leu Lys Ala Ala
   330 335 340
   acc agc ccg ttc tgg agt att ccg gga cgt att gca ccg ccg gaa gca 1290
   Thr Ser Pro Phe Trp Ser Ile Pro Gly Arg Ile Ala Pro Pro Glu Ala
   345 350 355
   gcc ggt agt gcc cgt ggt gta att aac gga ctg ggg aat ctg ggc ggt 1338
   Ala Gly Ser Ala Arg Gly Val Ile Asn Gly Leu Gly Asn Leu Gly Gly
   360 365 370 375
   ttc tgc ggc ccc tgg ctg gtc gga tta atg atc tac ctg tac gga cag 1386
   Phe Cys Gly Pro Trp Leu Val Gly Leu Met Ile Tyr Leu Tyr Gly Gln
   380 385 390
   aat gca gcc gtt gtt act ctg gca ggc tct ctg atc att gcc ggg att 1434
   Asn Ala Ala Val Val Thr Leu Ala Gly Ser Leu Ile Ile Ala Gly Ile
               395                 400                 405
   att gcg gca tta ctg cca acg cag tgt gat ctg cgc ccg gca gag gca      1482
   Ile Ala Ala Leu Leu Pro Thr Gln Cys Asp Leu Arg Pro Ala Glu Ala
           410                 415                 420
   cgg cag cag aat ttc acc cca cgt att cat gat gcc aaa taatactgtc       1531
   Arg Gln Gln Asn Phe Thr Pro Arg Ile His Asp Ala Lys
       425                 430                 435
   acccggtaac gctgttgccg ggtgcagcct tcacctttca gggcgtattt ttctgataac    1591
   cccgtgtaa                                                            1600
<210> 8
   <211> 436
   <212> PRT
   <213> Pantoea citrea
<400> 8
   Met Gln Lys Ser Gln Pro Gly Thr Arg Trp Phe Arg Ile Ile Val Pro
   1               5                   10                  15
   Ile Leu Ile Ala Cys Ile Met Ser Phe Met Asp Arg Val Asn Ile Ser
               20                  25                  30
   Phe Ala Leu Pro Gly Gly Met Glu Gln Asp Leu Leu Met Ser Ser Gln
           35                  40                  45
   Met Ala Gly Val Val Ser Gly Ile Phe Phe Ile Gly Tyr Leu Phe Leu
       50                  55                  60
   Gln Val Pro Gly Gly His Ile Ala Val Arg Gly Ser Gly Lys Arg Phe
   65                  70                  75                  80
   Ile Ala Trp Ser Leu Val Ala Trp Ala Val Val Ser Val Ala Thr Gly
                   85                  90                  95
   Phe Val Thr His Gln Tyr Gln Leu Leu Ile Leu Arg Phe Ala Leu Gly
               100                 105                 110
   Val Ser Glu Gly Gly Met Leu Pro Val Val Leu Thr Met Val Ser Asn
           115                 120                 125
   Trp Phe Pro Glu Lys Glu Leu Gly Arg Ala Asn Ala Phe Val Met Met
       130                 135                 140
   Phe Ala Pro Leu Gly Gly Met Ile Thr Ala Pro Val Ser Gly Trp Ile
   145                 150                 155                 160
   Ile Ala Leu Leu Asp Trp Arg Trp Leu Phe Ile Ile Glu Gly Leu Leu
                   165                 170                 175
   Ser Val Val Val Leu Ala Val Trp Trp Leu Met Val Ser Asp Arg Pro
               180                 185                 190
   Glu Asp Ala Arg Trp Leu Pro Ala Ala Glu Arg Glu Tyr Leu Leu Arg
           195                 200                 205
   Glu Met Ala Arg Asp Lys Ala Glu Arg Ser Lys Leu Pro Pro Ile Ser
       210                 215                 220
   His Ala Pro Leu Gln Glu Val Phe His Asn Pro Gly Leu Met Lys Leu
   225                 230                 235                 240
   Val Ile Leu Asn Phe Phe Tyr Gln Thr Gly Asp Tyr Gly Tyr Thr Leu
                   245                 250                 255
   Trp Leu Pro Thr Ile Ile Lys Asn Leu Thr Gly Ala Ser Ile Gly Asn
               260                 265                 270
   Val Gly Leu Leu Thr Val Leu Pro Phe Ile Ala Thr Leu Ser Gly Ile
           275                 280                 285
   Tyr Val Val Ser Tyr Leu Ser Asp Lys Thr Gly Lys Arg Arg Gln Trp
       290                 295                 300
   Val Met Ile Ser Leu Phe Cys Phe Ala Ala Cys Leu Leu Ala Ser Val
   305                 310                 315                 320
   Leu Leu Arg Glu Phe Val Leu Ala Ala Tyr Leu Ala Leu Val Ala Cys
                   325                 330                 335
   Gly Phe Phe Leu Lys Ala Ala Thr Ser Pro Phe Trp Ser Ile Pro Gly
               340                 345                 350
   Arg Ile Ala Pro Pro Glu Ala Ala Gly Ser Ala Arg Gly Val Ile Asn
           355                 360                 365
   Gly Leu Gly Asn Leu Gly Gly Phe Cys Gly Pro Trp Leu Val Gly Leu
       370                 375                 380
   Met Ile Tyr Leu Tyr Gly Gln Asn Ala Ala Val Val Thr Leu Ala Gly
   385                 390                 395                 400
   Ser Leu Ile Ile Ala Gly Ile Ile Ala Ala Leu Leu Pro Thr Gln Cys
                   405                 410                 415
   Asp Leu Arg Pro Ala Glu Ala Arg Gln Gln Asn Phe Thr Pro Arg Ile
               420                 425                 430
   His Asp Ala Lys
           435
<210> 9
   <211> 1500
   <212> DNA
   <213> Pantoea citrea
<220>
   <221> CDS
   <222> (154)...(1440)
<400> 9
   tcagctcagg cctcggattt cgttaacggt catgtcttat ttgtcgacgg cggaatgctg    60
   gcctcagtat aaaatacagg ggcagacgga atcagagttt gccctgaaga tatcttactg   120
   gttgcccctt cggcacacac aggatgttcc ccc atg aat aca agc aga aaa ctg    174
                                        Met Asn Thr Ser Arg Lys Leu
                                        1               5
   ccg gtg aaa cgc tgg tgg tat tta atg ccg gtg att ttt att act tac     222
   Pro Val Lys Arg Trp Trp Tyr Leu Met Pro Val Ile Phe Ile Thr Tyr
           10                  15                  20
   agc ctg gca tat ctg gat cgg gcc aac tac ggc ttt gct gct gcc tct     270
   Ser Leu Ala Tyr Leu Asp Arg Ala Asn Tyr Gly Phe Ala Ala Ala Ser
       25                  30                  35
   ggg att gaa gca gat ctt gga att agc cgt ggc acc tcc tct ctg att     318
   Gly Ile Glu Ala Asp Leu Gly Ile Ser Arg Gly Thr Ser Ser Leu Ile
   40                  45                  50                  55
   gga gca ctg ttc ttt ctc ggc tac ttc att ttt cag gtg ccc ggg gca     366
   Gly Ala Leu Phe Phe Leu Gly Tyr Phe Ile Phe Gln Val Pro Gly Ala
                   60                  65                  70
   att tat gca gtg aaa cgc agt gtc cgt aaa ctg gtg ttt acc agc ctg        414
   Ile Tyr Ala Val Lys Arg Ser Val Arg Lys Leu Val Phe Thr Ser Leu
                   75                  80                  85
   ctg ttg tgg gga ttt tgt gcc gct gcg acc gga ctt atc agc aat att        462
   Leu Leu Trp Gly Phe Cys Ala Ala Ala Thr Gly Leu Ile Ser Asn Ile
               90                  95                  100
   ccg gct ctg atg gtg atc cgc ttt gtt ctg ggt gtt gtt gaa gcc gca        510
   Pro Ala Leu Met Val Ile Arg Phe Val Leu Gly Val Val Glu Ala Ala
       105                 110                 115
   gtg atg cca gcg atg ctg att tac atc agc aac tgg ttc acc cgt cag        558
   Val Met Pro Ala Met Leu Ile Tyr Ile Ser Asn Trp Phe Thr Arg Gln
   120                 125                 130                 135
   gaa cgt tca cgg gct aat acc ttt ctg gta tta ggt aac ccg gtc acg        606
   Glu Arg Ser Arg Ala Asn Thr Phe Leu Val Leu Gly Asn Pro Val Thr
                   140                 145                 150
   gtg tta tgg atg tct att gtt tcc gga tat ctg atc aat gct ttt ggc        654
   Val Leu Trp Met Ser Ile Val Ser Gly Tyr Leu Ile Asn Ala Phe Gly
                   155                 160                 165
   tgg cgg gaa atg ttt att ttc gag ggt gtg cct gcc tta atc tgg gcc        702
   Trp Arg Glu Met Phe Ile Phe Glu Gly Val Pro Ala Leu Ile Trp Ala
               170                 175                 180
   atc ttc tgg tgg ttt att gtc cgg gac aaa ccg gag cag gtg agc tgg        750
   Ile Phe Trp Trp Phe Ile Val Arg Asp Lys Pro Glu Gln Val Ser Trp
       185                 190                 195
   ctg aca gaa aca gaa aag cag caa ctg gcc agt gca atg gct gaa gag        798
   Leu Thr Glu Thr Glu Lys Gln Gln Leu Ala Ser Ala Met Ala Glu Glu
   200                 205                 210                 215
   cag cag gca ata cca ccg atg cgc aat gtg ccg cag gcc ctg cgt tcc        846
   Gln Gln Ala Ile Pro Pro Met Arg Asn Val Pro Gln Ala Leu Arg Ser
                   220                 225                 230
   cgc aat gtg gtg gta ctg tgc ctg tta cac gct ctg tgg agc atc gga        894
   Arg Asn Val Val Val Leu Cys Leu Leu His Ala Leu Trp Ser Ile Gly
                   235                 240                 245
   gtg tat ggt ttt atg atg tgg atg cca tcg ata ctg cgt agc gct gca        942
   Val Tyr Gly Phe Met Met Trp Met Pro Ser Ile Leu Arg Ser Ala Ala
               250                 255                 260
   tca atg gac att gtc cgg gta ggc tgg ctg gcc gca gtt ccg tat ctg        990
   Ser Met Asp Ile Val Arg Val Gly Trp Leu Ala Ala Val Pro Tyr Leu
       265                 270                 275
   gcc gcg att att act atg ctg gtg att tca tgg ctg tca gat aaa acc       1038
   Ala Ala Ile Ile Thr Met Leu Val Ile Ser Trp Leu Ser Asp Lys Thr
   280                 285                 290                 295
   ggg ctg cgt cgg ctt ttt atc tgg cca tta ttg ctg att gcg tca gtt      1086
   Gly Leu Arg Arg Leu Phe Ile Trp Pro Leu Leu Leu Ile Ala Ser Val
                   300                 305                 310
   act ttt ttt ggg tcc tgg tta ctt ggg agc tac tca ttc tgg ttt tcc      1134
   Thr Phe Phe Gly Ser Trp Leu Leu Gly Ser Tyr Ser Phe Trp Phe Ser
               315                 320                 325
   tat ggc ttg ctg gta ctg gct gct gct tgt atg tat gcc ccg tat gga      1182
   Tyr Gly Leu Leu Val Leu Ala Ala Ala Cys Met Tyr Ala Pro Tyr Gly
           330                 335                 340
   ccg ttt ttt gcg ttg att cct gaa ttg ctg cca aaa aat gtg gcg ggg      1230
   Pro Phe Phe Ala Leu Ile Pro Glu Leu Leu Pro Lys Asn Val Ala Gly
       345                 350                 355
   att tct atc ggg tta att aac tgt tgc ggg gcg ctg gga gct ttt gcc      1278
   Ile Ser Ile Gly Leu Ile Asn Cys Cys Gly Ala Leu Gly Ala Phe Ala
   360                 365                 370                 375
   gga gcc tgg ctg gtg ggc tat ctt aat ggt ctg acc ggt ggt ccg ggg      1326
   Gly Ala Trp Leu Val Gly Tyr Leu Asn Gly Leu Thr Gly Gly Pro Gly
                   380                 385                 390
   gct tct tac act ttt atg gcc att gca ttg ctg gtt tct gta ggg ttg      1374
   Ala Ser Tyr Thr Phe Met Ala Ile Ala Leu Leu Val Ser Val Gly Leu
               395                 400                 405
   gtg ttt ttc ctg aaa gtc cct tca ggg aat ttg gtc act cgt cgg ttg      1422
   Val Phe Phe Leu Lys Val Pro Ser Gly Asn Leu Val Thr Arg Arg Leu
           410                 415                 420
   ctg aaa ggt gat gca aag taaaaggaat agcgatgaaa cggaacagga             1470
   Leu Lys Gly Asp Ala Lys
       425
   tgtctttgca ggatattgcg gacctcgccg                                     1500
<210> 10
   <211> 429
   <212> PRT
   <213> Pantoea citrea
<400> 10
   Met Asn Thr Ser Arg Lys Leu Pro Val Lys Arg Trp Trp Tyr Leu Met
    1               5                  10                  15
   Pro Val Ile Phe Ile Thr Tyr Ser Leu Ala Tyr Leu Asp Arg Ala Asn
               20                  25                  30
   Tyr Gly Phe Ala Ala Ala Ser Gly Ile Glu Ala Asp Leu Gly Ile Ser
           35                  40                  45
   Arg Gly Thr Ser Ser Leu Ile Gly Ala Leu Phe Phe Leu Gly Tyr Phe
       50                  55                  60
   Ile Phe Gln Val Pro Gly Ala Ile Tyr Ala Val Lys Arg Ser Val Arg
   65                  70                  75                  80
   Lys Leu Val Phe Thr Ser Leu Leu Leu Trp Gly Phe Cys Ala Ala Ala
                   85                  90                  95
   Thr Gly Leu Ile Ser Asn Ile Pro Ala Leu Met Val Ile Arg Phe Val
               100                 105                 110
   Leu Gly Val Val Glu Ala Ala Val Met Pro Ala Met Leu Ile Tyr Ile
           115                 120                 125
   Ser Asn Trp Phe Thr Arg Gln Glu Arg Ser Arg Ala Asn Thr Phe Leu
       130                 135                 140
   Val Leu Gly Asn Pro Val Thr Val Leu Trp Met Ser Ile Val Ser Gly
   145                 150                 155                 160
   Tyr Leu Ile Asn Ala Phe Gly Trp Arg Glu Met Phe Ile Phe Glu Gly
                   165                 170                 175
   Val Pro Ala Leu Ile Trp Ala Ile Phe Trp Trp Phe Ile Val Arg Asp
               180                 185                 190
   Lys Pro Glu Gln Val Ser Trp Leu Thr Glu Thr Glu Lys Gln Gln Leu
           195                 200                 205
   Ala Ser Ala Met Ala Glu Glu Gln Gln Ala Ile Pro Pro Met Arg Asn
       210                 215                 220
   Val Pro Gln Ala Leu Arg Ser Arg Asn Val Val Val Leu Cys Leu Leu
   225                 230                 235                 240
   His Ala Leu Trp Ser Ile Gly Val Tyr Gly Phe Met Met Trp Met Pro
                   245                 250                 255
   Ser Ile Leu Arg Ser Ala Ala Ser Met Asp Ile Val Arg Val Gly Trp
               260                 265                 270
   Leu Ala Ala Val Pro Tyr Leu Ala Ala Ile Ile Thr Met Leu Val Ile
           275                 280                 285
   Ser Trp Leu Ser Asp Lys Thr Gly Leu Arg Arg Leu Phe Ile Trp Pro
       290                 295                 300
   Leu Leu Leu Ile Ala Ser Val Thr Phe Phe Gly Ser Trp Leu Leu Gly
   305                 310                 315                 320
   Ser Tyr Ser Phe Trp Phe Ser Tyr Gly Leu Leu Val Leu Ala Ala Ala
                   325                 330                 335
   Cys Met Tyr Ala Pro Tyr Gly Pro Phe Phe Ala Leu Ile Pro Glu Leu
               340                 345                 350
   Leu Pro Lys Asn Val Ala Gly Ile Ser Ile Gly Leu Ile Asn Cys Cys
           355                 360                 365
   Gly Ala Leu Gly Ala Phe Ala Gly Ala Trp Leu Val Gly Tyr Leu Asn
       370                 375                 380
   Gly Leu Thr Gly Gly Pro Gly Ala Ser Tyr Thr Phe Met Ala Ile Ala
   385                 390                 395                 400
   Leu Leu Val Ser Val Gly Leu Val Phe Phe Leu Lys Val Pro Ser Gly
                   405                 410                 415
   Asn Leu Val Thr Arg Arg Leu Leu Lys Gly Asp Ala Lys
               420                 425
<210> 11
   <211> 1500
   <212> DNA
   <213> Klebsiella oxytoca
<220>
   <221> CDS
   <222> (70)...(1386)
<400> 11
   ctaaaacaag cacaataata ataatcacct tcatcaccag aatattttta atattacgag     60
   actataaag atg aat ata acc tct aac tct aca acc aaa gat ata ccg cgc    111
             Met Asn Ile Thr Ser Asn Ser Thr Thr Lys Asp Ile Pro Arg
              1               5                  10
   cag cgc tgg tta aga atc att ccg cct ata ctg atc act tgt att att      159
   Gln Arg Trp Leu Arg Ile Ile Pro Pro Ile Leu Ile Thr Cys Ile Ile
    15                  20                  25                  30
   tct tat atg gac cgg gtc aat att gcc ttt gcg atg ccc gga ggt atg      207
   Ser Tyr Met Asp Arg Val Asn Ile Ala Phe Ala Met Pro Gly Gly Met
                    35                  40                  45
   gat gcc gac tta ggt att tcc gcc acc atg gcg ggg ctg gcg ggc ggt      255
   Asp Ala Asp Leu Gly Ile Ser Ala Thr Met Ala Gly Leu Ala Gly Gly
                50                  55                  60
   att ttc ttt atc ggt tat cta ttt tta cag gtt ccc ggc ggg aaa att      303
   Ile Phe Phe Ile Gly Tyr Leu Phe Leu Gln Val Pro Gly Gly Lys Ile
            65                  70                  75
   gcc gtt cac ggt agc ggt aag aaa ttt atc ggc tgg tcg ctg gtc gcc      351
   Ala Val His Gly Ser Gly Lys Lys Phe Ile Gly Trp Ser Leu Val Ala
        80                  85                  90
   tgg gcg gtc atc tcc gtg ctg acg ggg tta att acc aat cag tac cag      399
   Trp Ala Val Ile Ser Val Leu Thr Gly Leu Ile Thr Asn Gln Tyr Gln
    95                 100                 105                 110
   ctg ctg gcc ctg cgc ttc tta ctg ggc gtg gcg gaa ggc ggt atg ctg      447
   Leu Leu Ala Leu Arg Phe Leu Leu Gly Val Ala Glu Gly Gly Met Leu
                   115                 120                 125
   ccg gtc gtt ctc acg atg atc agt aac tgg ttc ccc gac gct gaa cgc      495
   Pro Val Val Leu Thr Met Ile Ser Asn Trp Phe Pro Asp Ala Glu Arg
               130                 135                 140
   ggt cgc gcc aac gcg att gtc att atg ttt gtg ccg att gcc ggg att      543
   Gly Arg Ala Asn Ala Ile Val Ile Met Phe Val Pro Ile Ala Gly Ile
           145                 150                 155
   atc acc gcc cca ctc tca ggc tgg att atc acg gtt ctc gac tgg cgc      591
   Ile Thr Ala Pro Leu Ser Gly Trp Ile Ile Thr Val Leu Asp Trp Arg
       160                 165                 170
   tgg ctg ttt att atc gaa ggt ttg ctc tcg ctg gtt gtt ctg gtt ctg      639
   Trp Leu Phe Ile Ile Glu Gly Leu Leu Ser Leu Val Val Leu Val Leu
   175                 180                 185                 190
   tgg gca tac acc atc tat gac cgt ccg cag gaa gcg cgc tgg att tcc      687
   Trp Ala Tyr Thr Ile Tyr Asp Arg Pro Gln Glu Ala Arg Trp Ile Ser
                   195                 200                 205
   gaa gca gag aag cgc tat ctg gtc gag acg ctg gcc gcg gag caa aaa      735
   Glu Ala Glu Lys Arg Tyr Leu Val Glu Thr Leu Ala Ala Glu Gln Lys
               210                 215                 220
   gcc att gcc ggc acc gag gtg aaa aac gcc tct ctg agc gcc gtt ctc      783
   Ala Ile Ala Gly Thr Glu Val Lys Asn Ala Ser Leu Ser Ala Val Leu
           225                 230                 235
   tcc gac aaa acc atg tgg cag ctt atc gcc ctg aac ttc ttc tac cag      831
   Ser Asp Lys Thr Met Trp Gln Leu Ile Ala Leu Asn Phe Phe Tyr Gln
       240                 245                 250
   acc ggc att tac ggc tac acc ctg tgg cta ccc acc att ctg aaa gaa      879
   Thr Gly Ile Tyr Gly Tyr Thr Leu Trp Leu Pro Thr Ile Leu Lys Glu
   255                 260                 265                 270
   ttg acc cat agc agc atg ggg cag gtc ggc atg ctt gcc att ctg ccg      927
   Leu Thr His Ser Ser Met Gly Gln Val Gly Met Leu Ala Ile Leu Pro
                   275                 280                 285
   tac gtc ggc gcc att gct ggg atg ttc ctg ttt tcc tcc ctt tca gac      975
   Tyr Val Gly Ala Ile Ala Gly Met Phe Leu Phe Ser Ser Leu Ser Asp
               290                 295                 300
   cga acc ggt aaa cgc aag ctg ttc gtc tgc ctg ccg ctg att ggc ttc     1023
   Arg Thr Gly Lys Arg Lys Leu Phe Val Cys Leu Pro Leu Ile Gly Phe
           305                 310                 315
   gct ctg tgc atg ttc ctg tcg gtg gcg ctg aaa aac caa att tgg ctc     1071
   Ala Leu Cys Met Phe Leu Ser Val Ala Leu Lys Asn Gln Ile Trp Leu
       320                 325                 330
   tcc tat gcc gcg ctg gtc ggc tgc gga ttc ttc ctg caa tcg gcg gct     1119
   Ser Tyr Ala Ala Leu Val Gly Cys Gly Phe Phe Leu Gln Ser Ala Ala
   335                 340                 345                 350
   ggc gtg ttc tgg acc atc ccg gca cgt ctg ttc agc gcg gaa atg gcg     1167
   Gly Val Phe Trp Thr Ile Pro Ala Arg Leu Phe Ser Ala Glu Met Ala
                   355                 360                 365
   ggc ggc gcg cgc ggg gtt atc aac gcg ctt ggc aac ctc ggc gga ttt     1215
   Gly Gly Ala Arg Gly Val Ile Asn Ala Leu Gly Asn Leu Gly Gly Phe
               370                 375                 380
   tgt ggc cct tat gcg gtc ggg gtg ctg atc acg ttg tac agc aaa gac     1263
   Cys Gly Pro Tyr Ala Val Gly Val Leu Ile Thr Leu Tyr Ser Lys Asp
           385                 390                 395
   gct ggc gtc tat tgc ctg gcg atc tcc ctg gcg ctg gcc gcg ctg atg     1311
   Ala Gly Val Tyr Cys Leu Ala Ile Ser Leu Ala Leu Ala Ala Leu Met
       400                 405                 410
   gcg ctg ctg ctg ccg gcg aaa tgc gat gcc ggt gct gcg ccg gta aag     1359
   Ala Leu Leu Leu Pro Ala Lys Cys Asp Ala Gly Ala Ala Pro Val Lys
   415                 420                 425                 430
   acg ata aat cca cat aaa cgc act gcg taaactcgag cccggcggcg          1406
   Thr Ile Asn Pro His Lys Arg Thr Ala
                   435
   ctgcgcctgc cgggcctgcg aaatatgccg ggttcacccg gtaacaatga gatgcgaaag  1466
   atgagcaaga aacaggcctt ctggctgggt attg                              1500
<210> 12
   <211> 439
   <212> PRT
   <213> Klebsiella oxytoca
<400> 12
   Met Asn Ile Thr Ser Asn Ser Thr Thr Lys Asp Ile Pro Arg Gln Arg
    1               5                  10                  15
   Trp Leu Arg Ile Ile Pro Pro Ile Leu Ile Thr Cys Ile Ile Ser Tyr
               20                  25                  30
   Met Asp Arg Val Asn Ile Ala Phe Ala Met Pro Gly Gly Met Asp Ala
           35                  40                  45
   Asp Leu Gly Ile Ser Ala Thr Met Ala Gly Leu Ala Gly Gly Ile Phe
       50                  55                  60
   Phe Ile Gly Tyr Leu Phe Leu Gln Val Pro Gly Gly Lys Ile Ala Val
   65                  70                  75                  80
   His Gly Ser Gly Lys Lys Phe Ile Gly Trp Ser Leu Val Ala Trp Ala
                   85                  90                  95
   Val Ile Ser Val Leu Thr Gly Leu Ile Thr Asn Gln Tyr Gln Leu Leu
               100                 105                 110
   Ala Leu Arg Phe Leu Leu Gly Val Ala Glu Gly Gly Met Leu Pro Val
           115                 120                 125
   Val Leu Thr Met Ile Ser Asn Trp Phe Pro Asp Ala Glu Arg Gly Arg
       130                 135                 140
   Ala Asn Ala Ile Val Ile Met Phe Val Pro Ile Ala Gly Ile Ile Thr
   145                 150                 155                 160
   Ala Pro Leu Ser Gly Trp Ile Ile Thr Val Leu Asp Trp Arg Trp Leu
                   165                 170                 175
   Phe Ile Ile Glu Gly Leu Leu Ser Leu Val Val Leu Val Leu Trp Ala
               180                 185                 190
   Tyr Thr Ile Tyr Asp Arg Pro Gln Glu Ala Arg Trp Ile Ser Glu Ala
           195                 200                 205
   Glu Lys Arg Tyr Leu Val Glu Thr Leu Ala Ala Glu Gln Lys Ala Ile
       210                 215                 220
   Ala Gly Thr Glu Val Lys Asn Ala Ser Leu Ser Ala Val Leu Ser Asp
   225                 230                 235                 240
   Lys Thr Met Trp Gln Leu Ile Ala Leu Asn Phe Phe Tyr Gln Thr Gly
                   245                 250                 255
   Ile Tyr Gly Tyr Thr Leu Trp Leu Pro Thr Ile Leu Lys Glu Leu Thr
               260                 265                 270
   His Ser Ser Met Gly Gln Val Gly Met Leu Ala Ile Leu Pro Tyr Val
           275                 280                 285
   Gly Ala Ile Ala Gly Met Phe Leu Phe Ser Ser Leu Ser Asp Arg Thr
       290                 295                 300
   Gly Lys Arg Lys Leu Phe Val Cys Leu Pro Leu Ile Gly Phe Ala Leu
   305                 310                 315                 320
   Cys Met Phe Leu Ser Val Ala Leu Lys Asn Gln Ile Trp Leu Ser Tyr
                   325                 330                 335
   Ala Ala Leu Val Gly Cys Gly Phe Phe Leu Gln Ser Ala Ala Gly Val
               340                 345                 350
   Phe Trp Thr Ile Pro Ala Arg Leu Phe Ser Ala Glu Met Ala Gly Gly
           355                 360                 365
   Ala Arg Gly Val Ile Asn Ala Leu Gly Asn Leu Gly Gly Phe Cys Gly
       370                 375                 380
   Pro Tyr Ala Val Gly Val Leu Ile Thr Leu Tyr Ser Lys Asp Ala Gly
   385                 390                 395                 400
   Val Tyr Cys Leu Ala Ile Ser Leu Ala Leu Ala Ala Leu Met Ala Leu
                   405                 410                 415
   Leu Leu Pro Ala Lys Cys Asp Ala Gly Ala Ala Pro Val Lys Thr Ile
               420                 425                 430
   Asn Pro His Lys Arg Thr Ala
           435
<210> 13
   <211> 3153
   <212> DNA
   <213> Unknown
<220>
   <223> environmental source
<221> CDS
   <222> (292)...(1236)
<221> CDS
   <222> (1252)...(2280)
<221> CDS
   <222> (2293)...(3045)
<400> 13
   catgcctgca ggtcgactct agaggatctc gccgcgcctc aggtcgaggg atacactcgt     60
   cagcgctttc gtgccgccga actccttcga aacggcacga aactccagaa gtttgtccgt    120
   atccaccccg ctcctcccaa agctttatga ggctatagga tattgatatg gtatcgataa    180
   cactcctgtc aagaggcggt tttcacgcca ggcgggaggg caaaatagga ctggacaatt    240
   ccttcaagcg ggatatgtta tcgataacaa atcatcttcc ggaggagagc c atg agc     297
                                                            Met Ser
                                                            1
   aag atc gat gtg ttg cag gtc ggt ccc tac cct gca tgg gac gag gag      345
   Lys Ile Asp Val Leu Gln Val Gly Pro Tyr Pro Ala Trp Asp Glu Glu
           5                   10                  15
   cgc ctg aac gcg acc ttc acg atg cac cgc tat ttc gag gcg gcc gac      393
   Arg Leu Asn Ala Thr Phe Thr Met His Arg Tyr Phe Glu Ala Ala Asp
       20                  25                  30
   aag gcg gcg ttt ctg gcc gag cac ggc ggc acg atc cgc ggc atc gcc      441
   Lys Ala Ala Phe Leu Ala Glu His Gly Gly Thr Ile Arg Gly Ile Ala
   35                  40                  45                  50
   acg cgc ggc gag ctt ggt gcc aac cgg gcg atg atc gag gcg ctg ccg      489
   Thr Arg Gly Glu Leu Gly Ala Asn Arg Ala Met Ile Glu Ala Leu Pro
                   55                  60                  65
   aag ctg gaa gtg atc tcg gtc tac ggc gtc ggc ttc gat gcg gtg gac      537
   Lys Leu Glu Val Ile Ser Val Tyr Gly Val Gly Phe Asp Ala Val Asp
               70                  75                  80
   ctt tcg gcg gcc cgc gag cgc ggc atc cgc gtc acc aac acg ccc gac      585
   Leu Ser Ala Ala Arg Glu Arg Gly Ile Arg Val Thr Asn Thr Pro Asp
           85                  90                  95
   gtg ctc acc aag gac gtg gcc gat ctc ggc atc gcc atg atg ctg gcg      633
   Val Leu Thr Lys Asp Val Ala Asp Leu Gly Ile Ala Met Met Leu Ala
       100                 105                 110
   cag gcg cgc ggc gtc atc ggc gga gag gcc tgg gtg aag agc ggc gat      681
   Gln Ala Arg Gly Val Ile Gly Gly Glu Ala Trp Val Lys Ser Gly Asp
   115                 120                 125                 130
   tgg gca agc aag ggt ctc tat ccg ctg aag cgc cgc gta cat ggc atg      729
   Trp Ala Ser Lys Gly Leu Tyr Pro Leu Lys Arg Arg Val His Gly Met
                   135                 140                 145
   cgc gcc ggg gtg ctc ggc ctc ggc cgc atc ggc tac gag gtg gcc aag      777
   Arg Ala Gly Val Leu Gly Leu Gly Arg Ile Gly Tyr Glu Val Ala Lys
               150                 155                 160
   cgc ctt gcc ggc ttc gac atg gac atc gcc tac agc gac acc ggc ccg      825
   Arg Leu Ala Gly Phe Asp Met Asp Ile Ala Tyr Ser Asp Thr Gly Pro
           165                 170                 175
   aag gat ttc gcc agg gac tgg acc ttc gtc gcc gat ccg gcg gag ctg      873
   Lys Asp Phe Ala Arg Asp Trp Thr Phe Val Ala Asp Pro Ala Glu Leu
       180                 185                 190
   gcc gcc cgc tcc gac ttc ctc ttc gtc acg ctc gcc gcc tcc gcc gag      921
   Ala Ala Arg Ser Asp Phe Leu Phe Val Thr Leu Ala Ala Ser Ala Glu
   195                 200                 205                 210
   acg cgc cac atc gtc ggc cgc aag gtc atc gag gcg ctc ggc cct gag      969
   Thr Arg His Ile Val Gly Arg Lys Val Ile Glu Ala Leu Gly Pro Glu
                   215                 220                 225
   ggc atg ctg atc aac atc tcg cgc gct tcc aac atc gat gaa agc gcc     1017
   Gly Met Leu Ile Asn Ile Ser Arg Ala Ser Asn Ile Asp Glu Ser Ala
               230                 235                 240
   ctt ctc gac gcg ctg gag acg aag gcg ctc ggc tcg gcc gcg ctc gac     1065
   Leu Leu Asp Ala Leu Glu Thr Lys Ala Leu Gly Ser Ala Ala Leu Asp
           245                 250                 255
   gtc ttc gag ggc gag ccg aac ctc aat ccg cgt ttc ctt gcc ctc gac     1113
   Val Phe Glu Gly Glu Pro Asn Leu Asn Pro Arg Phe Leu Ala Leu Asp
       260                 265                 270
   aac gtc ctc ttg cag ccg cac atg gcc tcc ggc acg atc gag acc cgc      1161
   Asn Val Leu Leu Gln Pro His Met Ala Ser Gly Thr Ile Glu Thr Arg
   275                 280                 285                 290
   aag gcc atg ggc cag ctc gtc ttc gac aac ctg tcg gcc cat ttc gac      1209
   Lys Ala Met Gly Gln Leu Val Phe Asp Asn Leu Ser Ala His Phe Asp
                   295                 300                 305
   ggc cgg ccg ctg ccg acc ccg gtt ctg taaggagaga ggtcc atg aag gcg     1260
   Gly Arg Pro Leu Pro Thr Pro Val Leu                  Met Lys Ala
               310                 315
   atc gtc atc cat cag gcc aag gac ctg cgc gtc gag gac agc gcc gtc      1308
   Ile Val Ile His Gln Ala Lys Asp Leu Arg Val Glu Asp Ser Ala Val
       320                 325                 330
   gag gcg ccc ggc ccc ggc gag gtg gag atc cgc ctt gcc gcc ggc ggc      1356
   Glu Ala Pro Gly Pro Gly Glu Val Glu Ile Arg Leu Ala Ala Gly Gly
   335                 340                 345                 350
   atc tgc ggc tcg gac ctg cac tac tac aac cac ggc ggc ttc ggc acg      1404
   Ile Cys Gly Ser Asp Leu His Tyr Tyr Asn His Gly Gly Phe Gly Thr
                   355                 360                 365
   gtg cgc ctc aag gag ccg atg atc ctc ggc cat gag gtt tcc ggc cac      1452
   Val Arg Leu Lys Glu Pro Met Ile Leu Gly His Glu Val Ser Gly His
               370                 375                 380
   gtc gcg gcg ctc ggc gaa ggc gtc tcc ggc ctt gcc atc ggc gac ctc      1500
   Val Ala Ala Leu Gly Glu Gly Val Ser Gly Leu Ala Ile Gly Asp Leu
           385                 390                 395
   gtc gcc gtc tcg ccc tcg cgg ccc tgc ggg gcg tgc gac tat tgc ctc      1548
   Val Ala Val Ser Pro Ser Arg Pro Cys Gly Ala Cys Asp Tyr Cys Leu
       400                 405                 410
   aag ggc ttg gcg aac cat tgc ttc aac atg cgc ttc tac ggc tcg gcc      1596
   Lys Gly Leu Ala Asn His Cys Phe Asn Met Arg Phe Tyr Gly Ser Ala
   415                 420                 425                 430
   atg ccc ttc ccg cac atc cag ggc gcg ttc cgc gag cgg ctg gtc gcc      1644
   Met Pro Phe Pro His Ile Gln Gly Ala Phe Arg Glu Arg Leu Val Ala
                   435                 440                 445
   aag gcc agc cag tgc gtg aag gct gag ggc ctt tcg gca ggt gaa gcc      1692
   Lys Ala Ser Gln Cys Val Lys Ala Glu Gly Leu Ser Ala Gly Glu Ala
               450                 455                 460
   gcg atg gcc gag ccg ctc tcc gtc acg ctt cac gcc acg cgc cgg gcc      1740
   Ala Met Ala Glu Pro Leu Ser Val Thr Leu His Ala Thr Arg Arg Ala
           465                 470                 475
   ggc gaa atg ctg ggc aag cgc gtg ctc gtc acc ggc tgc ggg ccg atc      1788
   Gly Glu Met Leu Gly Lys Arg Val Leu Val Thr Gly Cys Gly Pro Ile
       480                 485                 490
   ggc acc ctg tcg atc ctc gcc gcc cgg cgc gcc ggc gcg gcg gag atc      1836
   Gly Thr Leu Ser Ile Leu Ala Ala Arg Arg Ala Gly Ala Ala Glu Ile
   495                 500                 505                 510
   gtc gcc gct gac ctt tcc gag cgt gca ctc ggc ttt gcc cgc gcc gtc      1884
   Val Ala Ala Asp Leu Ser Glu Arg Ala Leu Gly Phe Ala Arg Ala Val
                   515                 520                 525
   ggc gcg gac cgc acg gtc aac ctg tcg gaa gac cgc gac ggc ctc gtt      1932
   Gly Ala Asp Arg Thr Val Asn Leu Ser Glu Asp Arg Asp Gly Leu Val
               530                 535                 540
   ccg ttc agc gag aac aag gga tat ttc gat gtc ctc tac gaa tgc tcg      1980
   Pro Phe Ser Glu Asn Lys Gly Tyr Phe Asp Val Leu Tyr Glu Cys Ser
           545                 550                 555
   ggc gcc cag ccg gcg ctg gtt gcc ggc atc cag gcc ttg cgc ccg cgc      2028
   Gly Ala Gln Pro Ala Leu Val Ala Gly Ile Gln Ala Leu Arg Pro Arg
       560                 565                 570
   ggc gtc atc gtc cag ctc ggc ctc ggc ggc gag atg agc ctt ccc atg      2076
   Gly Val Ile Val Gln Leu Gly Leu Gly Gly Glu Met Ser Leu Pro Met
   575                 580                 585                 590
   atg gcg atc acc gcc aag gaa ctg gac ctg cgc ggc tcc ttc cgc ttc      2124
   Met Ala Ile Thr Ala Lys Glu Leu Asp Leu Arg Gly Ser Phe Arg Phe
                   595                 600                 605
   cat gag gaa ttc gcc gtc gcc gtg aag ctg atg cag ggc ggc ctc atc      2172
   His Glu Glu Phe Ala Val Ala Val Lys Leu Met Gln Gly Gly Leu Ile
               610                 615                 620
   gac gtg aag ccg ctg atc acc cat act ttg ccg ctt gcc gat gcg ctt      2220
   Asp Val Lys Pro Leu Ile Thr His Thr Leu Pro Leu Ala Asp Ala Leu
           625                 630                 635
   cag gcc ttc gag atc gcc tcg gac aag ggg caa tcg atg aag act cag      2268
   Gln Ala Phe Glu Ile Ala Ser Asp Lys Gly Gln Ser Met Lys Thr Gln
       640                 645                 650
   atc gca ttc agt taaggaagag cc atg agc atc cag ctt ttc gac ctc acg    2319
   Ile Ala Phe Ser                Met Ser Ile Gln Leu Phe Asp Leu Thr
   655                                 660                 665
   ggc aag cgc gcc ctc gtc acc ggc tcc tcg cag ggt atc ggc tat gcg      2367
   Gly Lys Arg Ala Leu Val Thr Gly Ser Ser Gln Gly Ile Gly Tyr Ala
           670                 675                 680
   ctc gcc aag ggc ctt gcc gcc gcc ggc gcg gac atc gtc ctc aac ggc      2415
   Leu Ala Lys Gly Leu Ala Ala Ala Gly Ala Asp Ile Val Leu Asn Gly
       685                 690                 695
   cgc gac gcg gcc aag ctg gcg gcc gcg gcg cag gaa ctc ggc gca aag      2463
   Arg Asp Ala Ala Lys Leu Ala Ala Ala Ala Gln Glu Leu Gly Ala Lys
   700                 705                 710                 715
   cac acg ctc gcc ttc gac gcc acc gac cat gcc gcc gtg cgc gcg gcc      2511
   His Thr Leu Ala Phe Asp Ala Thr Asp His Ala Ala Val Arg Ala Ala
                   720                 725                 730
   atc gac gcc ttc gag gcg gag gtc ggc ccc atc gac atc ctc gtc aac      2559
   Ile Asp Ala Phe Glu Ala Glu Val Gly Pro Ile Asp Ile Leu Val Asn
               735                 740                 745
   aat gcc ggc atg cag cac cgc acg ccg ctg gag gat ttc ccc gcc gat      2607
   Asn Ala Gly Met Gln His Arg Thr Pro Leu Glu Asp Phe Pro Ala Asp
           750                 755                 760
   gcc ttc gag cgc atc ctg aag acc aac atc tcg acg gtc ttc aat gtc      2655
   Ala Phe Glu Arg Ile Leu Lys Thr Asn Ile Ser Thr Val Phe Asn Val
       765                 770                 775
   ggc cag gcc gtc gcg cgc cac atg atc gcg cgc ggc gcg ggc aag atc      2703
   Gly Gln Ala Val Ala Arg His Met Ile Ala Arg Gly Ala Gly Lys Ile
   780                 785                 790                 795
   atc aac atc gcc agc gtg cag acc gcg ctc gcc cgc ccc ggc atc gcg      2751
   Ile Asn Ile Ala Ser Val Gln Thr Ala Leu Ala Arg Pro Gly Ile Ala
                   800                 805                 810
   ccc tat acc gcc acc aag ggc gcc gtc ggc aac ctc acc aag ggc atg      2799
   Pro Tyr Thr Ala Thr Lys Gly Ala Val Gly Asn Leu Thr Lys Gly Met
               815                 820                 825
   gcg acc gac tgg gcg aaa tac ggc ctg caa tgc aac gcc atc gcg ccg      2847
   Ala Thr Asp Trp Ala Lys Tyr Gly Leu Gln Cys Asn Ala Ile Ala Pro
           830                 835                 840
   ggc tat ttc gac acg ccg ctc aat gcc gcg ctg gtc gcc gat ccg gcc      2895
   Gly Tyr Phe Asp Thr Pro Leu Asn Ala Ala Leu Val Ala Asp Pro Ala
       845                 850                 855
   ttt tcc gcc tgg ctg gaa aag cgc acg ccg gcc ggc cgc tgg ggc aag      2943
   Phe Ser Ala Trp Leu Glu Lys Arg Thr Pro Ala Gly Arg Trp Gly Lys
   860                 865                 870                 875
   gtg gag gag ctg atc ggc gcc tgc atc ttt ctt tcc tcc gac gct tcc      2991
   Val Glu Glu Leu Ile Gly Ala Cys Ile Phe Leu Ser Ser Asp Ala Ser
                   880                 885                 890
   tcc ttc gtg aac gga cac acg ctc tat gtc gac ggc ggc atc acg gcc      3039
   Ser Phe Val Asn Gly His Thr Leu Tyr Val Asp Gly Gly Ile Thr Ala
               895                 900                 905
   tcg ctc tgaggacaac aggcgcatcg tcctgatggg cgtcgccggc tgcggcaagt      3095
   Ser Leu
   ccgccgtcgg cgcggcgctc gccgcgcggc tcggtgcgat ccccgggtac cgagctcg      3153
<210> 14
   <211> 315
   <212> PRT
   <213> Unknown
<220>
   <223> environmental source
<400> 14
   Met Ser Lys Ile Asp Val Leu Gln Val Gly Pro Tyr Pro Ala Trp Asp
    1               5                  10                  15
   Glu Glu Arg Leu Asn Ala Thr Phe Thr Met His Arg Tyr Phe Glu Ala
               20                  25                  30
   Ala Asp Lys Ala Ala Phe Leu Ala Glu His Gly Gly Thr Ile Arg Gly
           35                  40                  45
   Ile Ala Thr Arg Gly Glu Leu Gly Ala Asn Arg Ala Met Ile Glu Ala
       50                  55                  60
   Leu Pro Lys Leu Glu Val Ile Ser Val Tyr Gly Val Gly Phe Asp Ala
   65                  70                  75                  80
   Val Asp Leu Ser Ala Ala Arg Glu Arg Gly Ile Arg Val Thr Asn Thr
                   85                  90                  95
   Pro Asp Val Leu Thr Lys Asp Val Ala Asp Leu Gly Ile Ala Met Met
               100                 105                 110
   Leu Ala Gln Ala Arg Gly Val Ile Gly Gly Glu Ala Trp Val Lys Ser
           115                 120                 125
   Gly Asp Trp Ala Ser Lys Gly Leu Tyr Pro Leu Lys Arg Arg Val His
       130                 135                 140
   Gly Met Arg Ala Gly Val Leu Gly Leu Gly Arg Ile Gly Tyr Glu Val
   145                 150                 155                 160
   Ala Lys Arg Leu Ala Gly Phe Asp Met Asp Ile Ala Tyr Ser Asp Thr
                   165                 170                 175
   Gly Pro Lys Asp Phe Ala Arg Asp Trp Thr Phe Val Ala Asp Pro Ala
               180                 185                 190
   Glu Leu Ala Ala Arg Ser Asp Phe Leu Phe Val Thr Leu Ala Ala Ser
           195                 200                 205
   Ala Glu Thr Arg His Ile Val Gly Arg Lys Val Ile Glu Ala Leu Gly
       210                 215                 220
   Pro Glu Gly Met Leu Ile Asn Ile Ser Arg Ala Ser Asn Ile Asp Glu
   225                 230                 235                 240
   Ser Ala Leu Leu Asp Ala Leu Glu Thr Lys Ala Leu Gly Ser Ala Ala
                   245                 250                 255
   Leu Asp Val Phe Glu Gly Glu Pro Asn Leu Asn Pro Arg Phe Leu Ala
               260                 265                 270
   Leu Asp Asn Val Leu Leu Gln Pro His Met Ala Ser Gly Thr Ile Glu
           275                 280                 285
   Thr Arg Lys Ala Met Gly Gln Leu Val Phe Asp Asn Leu Ser Ala His
       290                 295                 300
   Phe Asp Gly Arg Pro Leu Pro Thr Pro Val Leu
   305                 310                 315
<210> 15
   <211> 343
   <212> PRT
   <213> Unknown
<220>
   <223> environmental source
<400> 15
   Met Lys Ala Ile Val Ile His Gln Ala Lys Asp Leu Arg Val Glu Asp
   1               5                   10                  15
   Ser Ala Val Glu Ala Pro Gly Pro Gly Glu Val Glu Ile Arg Leu Ala
               20                  25                  30
   Ala Gly Gly Ile Cys Gly Ser Asp Leu His Tyr Tyr Asn His Gly Gly
           35                  40                  45
   Phe Gly Thr Val Arg Leu Lys Glu Pro Met Ile Leu Gly His Glu Val
       50                  55                  60
   Ser Gly His Val Ala Ala Leu Gly Glu Gly Val Ser Gly Leu Ala Ile
   65                  70                  75                  80
   Gly Asp Leu Val Ala Val Ser Pro Ser Arg Pro Cys Gly Ala Cys Asp
                   85                  90                  95
   Tyr Cys Leu Lys Gly Leu Ala Asn His Cys Phe Asn Met Arg Phe Tyr
               100                 105                 110
   Gly Ser Ala Met Pro Phe Pro His Ile Gln Gly Ala Phe Arg Glu Arg
           115                 120                 125
   Leu Val Ala Lys Ala Ser Gln Cys Val Lys Ala Glu Gly Leu Ser Ala
       130                 135                 140
   Gly Glu Ala Ala Met Ala Glu Pro Leu Ser Val Thr Leu His Ala Thr
   145                 150                 155                 160
   Arg Arg Ala Gly Glu Met Leu Gly Lys Arg Val Leu Val Thr Gly Cys
                   165                 170                 175
   Gly Pro Ile Gly Thr Leu Ser Ile Leu Ala Ala Arg Arg Ala Gly Ala
               180                 185                 190
   Ala Glu Ile Val Ala Ala Asp Leu Ser Glu Arg Ala Leu Gly Phe Ala
           195                 200                 205
   Arg Ala Val Gly Ala Asp Arg Thr Val Asn Leu Ser Glu Asp Arg Asp
       210                 215                 220
   Gly Leu Val Pro Phe Ser Glu Asn Lys Gly Tyr Phe Asp Val Leu Tyr
   225                 230                 235                 240
   Glu Cys Ser Gly Ala Gln Pro Ala Leu Val Ala Gly Ile Gln Ala Leu
                   245                 250                 255
   Arg Pro Arg Gly Val Ile Val Gln Leu Gly Leu Gly Gly Glu Met Ser
               260                 265                 270
   Leu Pro Met Met Ala Ile Thr Ala Lys Glu Leu Asp Leu Arg Gly Ser
           275                 280                 285
   Phe Arg Phe His Glu Glu Phe Ala Val Ala Val Lys Leu Met Gln Gly
       290                 295                 300
   Gly Leu Ile Asp Val Lys Pro Leu Ile Thr His Thr Leu Pro Leu Ala
   305                 310                 315                 320
   Asp Ala Leu Gln Ala Phe Glu Ile Ala Ser Asp Lys Gly Gln Ser Met
                   325                 330                 335
   Lys Thr Gln Ile Ala Phe Ser
               340
<210> 16
   <211> 251
   <212> PRT
   <213> Unknown
<220>
   <223> environmental source
<400> 16
   Met Ser Ile Gln Leu Phe Asp Leu Thr Gly Lys Arg Ala Leu Val Thr
    1               5                  10                  15
   Gly Ser Ser Gln Gly Ile Gly Tyr Ala Leu Ala Lys Gly Leu Ala Ala
               20                  25                  30
   Ala Gly Ala Asp Ile Val Leu Asn Gly Arg Asp Ala Ala Lys Leu Ala
           35                  40                  45
   Ala Ala Ala Gln Glu Leu Gly Ala Lys His Thr Leu Ala Phe Asp Ala
       50                  55                  60
   Thr Asp His Ala Ala Val Arg Ala Ala Ile Asp Ala Phe Glu Ala Glu
   65                  70                  75                  80
   Val Gly Pro Ile Asp Ile Leu Val Asn Asn Ala Gly Met Gln His Arg
                   85                  90                  95
   Thr Pro Leu Glu Asp Phe Pro Ala Asp Ala Phe Glu Arg Ile Leu Lys
               100                 105                 110
   Thr Asn Ile Ser Thr Val Phe Asn Val Gly Gln Ala Val Ala Arg His
           115                 120                 125
   Met Ile Ala Arg Gly Ala Gly Lys Ile Ile Asn Ile Ala Ser Val Gln
       130                 135                 140
   Thr Ala Leu Ala Arg Pro Gly Ile Ala Pro Tyr Thr Ala Thr Lys Gly
   145                 150                 155                 160
   Ala Val Gly Asn Leu Thr Lys Gly Met Ala Thr Asp Trp Ala Lys Tyr
                   165                 170                 175
   Gly Leu Gln Cys Asn Ala Ile Ala Pro Gly Tyr Phe Asp Thr Pro Leu
               180                 185                 190
   Asn Ala Ala Leu Val Ala Asp Pro Ala Phe Ser Ala Trp Leu Glu Lys
           195                 200                 205
   Arg Thr Pro Ala Gly Arg Trp Gly Lys Val Glu Glu Leu Ile Gly Ala
       210                 215                 220
   Cys Ile Phe Leu Ser Ser Asp Ala Ser Ser Phe Val Asn Gly His Thr
   225                 230                 235                 240
   Leu Tyr Val Asp Gly Gly Ile Thr Ala Ser Leu
                   245                 250
<210> 17
   <211> 32
   <212> DNA
   <213> Artificial Sequence
<220>
   <223> synthetic construct
<400> 17
   acccaagctt caccaaaaga gtgaagagga ag 32
<210> 18
   <211> 34
   <212> DNA
   <213> Artificial Sequence
<220>
   <223> synthetic construct
<400> 18
   cgtatctaga aaaatattct ggtgatgaag gtga 34
<210> 19
   <211> 34
   <212> DNA
   <213> Artificial Sequence
<220>
   <223> synthetic construct
<400> 19
   agactctaga tccacataaa cgcactgcgt aaac 34
<210> 20
   <211> 33
   <212> DNA
   <213> Artificial Sequence
<220>
   <223> synthetic construct
<400> 20
   gaggggatcc tggcttcgtg aacgatatac tgg 33
<210> 21
   <211> 31
   <212> DNA
   <213> Artificial Sequence
<220>
   <223> synthetic construct
<400> 21
   aataggatcc ttcatcacca gaatattttt a 31
<210> 22
   <211> 28
   <212> DNA
   <213> Artificial Sequence
<220>
   <223> synthetic construct
<400> 22
   cataggtacc ggctttcaga taggtgcc 28